Europäisches Patentamt

European Patent Office

Office européen des brevets

(11) Publication number: **0 418 972 A1**

# EUROPEAN PATENT APPLICATION

(21) Application number: 90202471.0

(22) Date of filing: **18.09.90**

(51) Int. Cl.5: **C07C 2/84**, B01J 23/02, B01J 27/232

(30) Priority: **19.09.89 US 409359**
**26.06.90 US 543393**

(43) Date of publication of application:
**27.03.91 Bulletin 91/13**

(84) Designated Contracting States:
**AT BE CH DE DK ES FR GB GR IT LI LU NL SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **Warren, Barbara Knight**
**1620 Woodvale Drive**
**Charleston, West Virginia 25314(US)**
Inventor: **Chafin, Richard Barry**
**144 Washington Circle**
**Hurrican, West Virginia 25526(US)**

(74) Representative: **Smulders, Theodorus A.H.J., Ir. et al**
**Vereenigde Octrooibureaux Nieuwe Parklaan 107**
**NL-2587 BP 's-Gravenhage(NL)**

(54) Strontium and/or barium-containing catalysts for oxidative coupling processes.

(57) Oxidative coupling of lower alkane to higher hydrocarbon is conducted using supported catalyst comprising at least one of barium hydroxide, strontium hydroxide, barium and strontium carbonate in the presence of halogen component. High yields of higher hydrocarbon can be achieved with long catalyst life.

EP 0 418 972 A1

## STRONTIUM AND/OR BARIUM-CONTAINING CATALYSTS FOR OXIDATIVE COUPLING PROCESSES

The invention was made under United States of America Government support under Contract No. DE-AC22-87PC79817 awarded by the Department of Energy. The Government has certain rights in this invention.

This invention relates to processes for the oxidative coupling of lower molecular weight alkane to higher molecular weight hydrocarbon in which the catalysts exhibit enhanced stability.

Background of the Invention

Processes for the conversion of lower molecular weight alkanes such as methane to higher molecular weight hydrocarbons which have greater value are sought. One of the proposals for the conversion of lower molecular weight alkanes is by oxidative coupling. For instance, G. E. Keller and M. M. Bhasin disclose in Journal of Catalysis , Volume 73, pages 9 to 19 (1982) that methane can be converted to, e.g., ethylene. The publication by Keller, et al., has preceded the advent of substantial patent and open literature disclosures by numerous researchers pertaining to processes for the oxidative coupling of lover alkanes and catalysts for such processes.

In order for an oxidative coupling process to be commercially attractive the process should be capable of providing a good rote of conversion of the lower alkanes with high selectivity to the sought higher molecular weight hydrocarbons. Since conversion and selectivity can be enhanced by catalysts, catalytic processes have been the thrust of work done by researchers in oxidative coupling.

No general types of oxidative coupling processes are the sequential, or pulsed, processes and the cofeed processes. The sequential processes are characterized by alternately cycling an oxygen-containing gas and an alkane-containing gas for contact with catalyst. These processes typically provide high selectivities to higher hydrocarbon but suffer from operational complexities in cycling the catalyst environment and in the tendency of the processes to produce less desirable, higher molecular weight products and to have carbonaceous deposits form on the catalyst, thereby necessitating regeneration. Thus, from an operational standpoint, cofeed processes, i.e., processes, in which oxygen-containing material and alkane are simultaneously fed to the reaction zone containing the catalyst, are more desirable.

In order for cofeed processes to be commercially attractive, especially for the production of large volume commodity chemicals such as ethylene and ethane ($C_2$'s), not only should the conversion of alkane be high and the selectivity to higher hydrocarbons as opposed to combustion products such as carbon dioxide and carbon monoxide be high, but also, the catalyst should exhibit a relatively long lifetime with high performance. In many instances, the literature and patents describing oxidative coupling processes and catalysts do not relate experiences with catalyst stability. However, due to the lifetime problems that have plagued oxidative coupling catalysts, skepticism appears to exist that oxidative coupling catalysts exhibit long useful lifetimes absent empirical demonstrations.

Numerous catalysts have been proposed by researchers for oxidative coupling processes. These catalyst have included catalysts containing alkali and/or alkaline earth metals. The alkali and alkaline earth metals have been suggested as being in the oxide, carbonate and halide forms. Other components such as rhenium, tungsten, copper, bismuth, lead, tin, iron, nickel, zinc, indium, vanadium, palladium, platinum, iridium, uranium, osmium, rhodium, zirconium, titanium, lanthanum, aluminum, chromium, cobalt, beryllium, germanium, antimony, gallium, manganese, yttrium, cerium, praseodymium (and other rare earth oxides), scandium, molybdenum, thallium, thorium, cadmium, boron, among other components, have also been suggested for use in oxidative coupling catalysts. See, for instance, United States patents Nos. 4,450,310; 4,443,646; 4,499,324; 4,443,645; 4,443,648; 4,172,810; 4,205,194; 4,239,658; 4,523,050; 4,443,647; 4,499,323; 4,443,644; 4,444,984; 4,695,668; 4,704,487; 4,777,313; 4,780,449; International Patent publication NO 86/07351, European Patent Application publications Nos. 189079 (1986); 206042 (1986); 206044 (1986) and 177327 (1985), Australian Patent No. 52925 (1986), Moriyama, et al., "Oxidative Dimerization of Methane Over Promoted MgO, Important Factors," Chem. Sac. Japan, Chem. Lett., 1165 (1986), and Emesh, et al., "Oxidative coupling of Methane over the Oxides of Groups IIIA, IVA and VA Metals," J. Phys. Chem, Vol. 90, 4785 (1986).

Several researchers have proposed the use of alkali or alkaline earth metals in the form of halides (e.g., chloride, bromide or iodide) in oxidative coupling catalysts. Australian Patent No. 52925 discloses the use of supported calcium chloride, barium bromide, potassium iodide, lithium chloride, cesium chloride, among others for catalysts to oxidatively couple methane to ethane and ethylene. The catalysts are supported. The

2

EP 0 418 972 A1

patent application states at page 5,

"The chloride, bromide and iodide catalysts are preferably employed on a support consisting of pumice-stone, silica gel, kieselghur, precipitated silica and/or oxides of the alkaline earth elements and/or alumina oxide, silicon dioxide, zinc oxide, titanium dioxide, zirconium dioxide and/or silicon carbide. Of the oxides of alkaline earth elements which are employed as a support, magnesium oxide and calcium oxide are preferred."

The patentees disclose feeding hydrogen halide to the reaction zone. None of the reported examples use the addition of hydrogen halide. Also, the patentees did not report the time on stream of the catalyst when the conversion values are determined. Metal halide catalysts often decompose upon initiation of the process. This produces an initial unsteady state which can provide exceptionally high selectivities to $C_2$'s and high ethylene to ethane ratios. See also the corresponding German patent application 3,503,664. Included within the examples are illustrations of the use of, e.g., $CaCl_2$ on pumice, $CaCl_2$-$MgCl_2$ on pumice, BaBr on pumice, $CaI_2$ on pumice, $BaBr_2$ on calcium oxide, $CaBr_2$ on magnesia, $BaBr_2$ on silicon carbide, $BaBr_2$ on zinc oxide, and others.

The deactivation of oxidation coupling catalysts through the loss of components via vaporization of halide compounds under the high temperatures of oxidative coupling has been opined by Fujimoto, et al., "Oxidative coupling of Methane with Alkaline Earth Halides Catalysts Supported on Alkaline Earth Oxide", Chem. Soc. Japan, Chem. Lett., 2157 (1987). Catalysts exemplified include $MgCl_2$ on titania, $MgF_2$ on magnesia, $MgCl_2$ on magnesia, $CaCl_2$ on magnesia, $MgCl_2$ on calcium oxide, $CaF_2$ on calcium oxide, $CaBr_2$ on calcium oxide, $MgCl_2$ on silica, $MgBr_2$ on magnesia, CaBr on magnesia, $MgBr_2$ on calcium oxide and $CaCl_2$ on calcium oxide.

European Patent Application 210 383 (1986) discloses the addition of gas phase material containing halogen component such as chlorine, methyl chloride and methyl dichloride. Enhanced selectivities are reported when the halogen component is present. The catalysts include those containing one or more of alkali and alkaline earth metal, alkali metal with lanthanide oxide, zinc oxide, titanium oxide or zirconium oxide, and others. United States Patent No. 4,654,460 discloses the addition of a halogen-containing material either in the catalyst or via a feed gas in an oxidative coupling process. The catalyst contains one or more alkali metal and alkaline earth metal components. Although no working examples are provided illustrating the effect of halide additions, conversions of methane are said to be increased with halides and, selectivities to higher hydrocarbons, particularly ethylene, improved. See also, Burch, et al., "Role of Chlorine in Improving Selectivity in the Oxidative Coupling of Methane to Ethylene", Appl. Catal., Vol. 46, 69 (1989) and "The Importance of Heterogeneous and Homogeneous Reactions in Oxidative Coupling of Methane Over Chloride Promoted Oxide Catalysts," Catal. Lett., vol. 2, 249 (1989), who propose mechanistic possibilities for the effect of halide in oxidative coupling of methane, and Minachev, et al., "Oxidative Condensations of Methane- a New Pathway to the Synthesis of Ethane, Ethylene, and Other Hydrocarbons", Russ. Chem. Rev., Vol. 57, 221 (1988).

Barium-containing catalysts for oxidative coupling have been proposed by, for instance, Yamagata, et al., "Oxidative Coupling of Methane over BaO Mixed with CaO and MgO," Chem. Soc. Japan, Chem. Lett., 81 (1987) (catalysts include $BaO/MgO$, $SaO/Al_2O_3$, $Bao/CaO$, $BaO/ZrO_2$ and $BaO/TiO_2$). The authors conclude that the $SaO/MgO$ and $BaO/CaO$ catalysts are much more effective than BaO on other supports such as titania, zirconia, alumina, silica and ferric oxide. They said that XRD analyses suggest the formation of some mixed compounds, probably the mixed oxides on the mixed carbonates, together with $BaCO_3$.

Aika, et al., "Oxidative Dimerization of Methane over $BaCO_3$, $SrCO_3$ and these Catalysts Promoted with Alkali", J. Chem. Soc., Chem. Comm. 1210 (1986) (catalysts exemplified include $BaCO_3$, BaO, $SrCO_3$, $TiO_2$, $ZrO_2$), state:

"Oxides of alkali earth elements are more effective than the other oxides studied here. The effectiveness of these catalysts increases with increasing atomic number ($BaO>SrO>CaO>MgO$)." "However, BaO and SrO gave no $CO_2$ as a product during several hours of the reaction because these oxides are partly converted into their carbonates under reaction conditions."..."Another problem with BaO and SrO is the corrosion of the glass reactor."

"In order to overcome these problems, we studied oxidative dimerization over $BaCO_3$ and $SrCO_3$. The activities and the kinetic behavior of these carbonates are almost identical to those of their oxides." (p 1210-1211).

See also, International Patent Application WO 86/7351, ($BaO/La_2O_3$, $BaO/MgO$); U.S. Patents Nos. 4,172,810 ($Ba$-$Cr$-$Pt/MgAl_2O_4$, sequential process), 4,704,487, 4,704,488, and 4,709,108 ($BaO/Al_2O_3$); Nagamoto, et al., "Methane Oxidation over Perovskite-Type Oxide containing Alkaline Earth Metal", Chem. Soc. Japan, Chem. Lett. 237 (1988), ($SrZrO_3$, $BaTiO_3$); Otsuko, et al., "Peroxide Anions as Possible Active Species in Oxidative Coupling of Methane", Chem. Soc. Japan, Chem. Lett., 77 (1987) (Strontium peroxide

3

and barium peroxide mechanisms); Roos, et al., "Selective Oxidation of Methane to Ethane and Ethylene Over Various Oxide catalysts", Catal. Today, Vol. 1, 133 (1987), ($Ca/Al_2O_3$); Iwamatsu, et al., "Importance of the Specific Surface Area in Oxidative Dimerization of Methane over Promoted MgO", J. CHem. Soc., Chem. Comm., 19 (1987), (Sr/MgO, Ca/MgO, Ba/MgO); Moriyama, et al., "Oxidative Dimerization of Methane over Promoted MgO. Important Factors", Chem. Soc. Japan, Chem. Lett., 1165 (1986) (Ba/MgO, Sr/MgO, Ca/MgO); Roos, et al., "Oxidative Coupling of Methane; Catalyst Requirements Process Conditions", Studies in Surface Science & Catalysis; #36 Methane Conversion Symp., Auckland, New Zealand, April, 1987, 373, (Ba/MgO); and United States Patent No. 4,780,449 ($BaO/La_2O_3$, $SrO/La_2O_3$); and Aika, et al., "Oxidative Dimerization of Methane over $BaCO_3$, $SrCO_3$ and these Catalysts Promoted with Alkali", J. Chem. Soc., Chem. Comm. 1210 (1986) (catalysts exemplified include $BaCO_3$, BaO, $SrCO_3$, $TiO_2$, $ZrO_2$).

Aika, et al., do not disclose or suggest the use of a halogen component. Minachev, et al., supra, report catalytic properties of numerous catalysts in a review of the literature, including SrO, $SrCO_3$, BaO, $BaO/Al_2O_3$ and $BaCO_3$. They state in connection with halide salt catalysts:

"Thus the efficiency of halogen-containing catalysts in the oxidative condensation is greater than that of other catalytic systems." (p. 227)

Carreiro, et al., "Oxidative Coupling of Methane", Jour. of Catal., vol. 117, 258 (1989) studied various oxidative coupling catalysts. They report

"Although SrO and BaO appeared rather attractive from a selectivity point of view... they were not chemically stable during the oxidative coupling of methane: extended exposure to $H_2O$ vapor formed during the oxidative methane conversion resulted in the formation of the respective hydroxides and successively of hydrated hydroxides having melting points below the reaction temperature applied. The undesired hydration reaction could not be avoided. Furthermore, carbonates were formed. X-ray diffraction analysis of the SrO catalyst after exposure to the catalytic reaction showed the following phase composition: $Sr(OH)_2 \cdot H_2O$, Sr-$(OH)_2$, $SrCO_3$, and SrO. Tentatively, it may be assumed that liquid hydroxides are stabilized in a solid carbonate matrix similarly to supported liquid-phase catalysts. Since this behavior of SrO and BaO catalysts was detrimental to catalyst operation over an extended period of time these catalysts were not included in any further studies." (p. 260).

The authors reported SrO and BaO to have similar selectivities to $C_2$'s and $SrCO_3$ to be inferior to SrO. The catalysts were not supported nor were any halogen-containing compounds provided to the catalysts.

## Summary of the Invention

By this invention processes are provided for the oxidative coupling of lower alkane to produce heavier hydrocarbon which provide not only good performance, i.e., conversion of alkane and selectivity to higher hydrocarbon, but also are capable of retaining the good performance over extended periods of operation. In accordance with this invention, the lifetime of a strontium and/or barium-containing catalyst in the cofeed oxidative coupling process can be enhanced by providing strontium and/or barium as hydroxide (including hydrated oxide) and/or carbonate salt on a support and feeding (intermittently or continuously) sufficient halogencontaining vapor phase additive to enhance the stability of the catalyst during oxidative coupling. The hydroxide and/or carbonate-containing catalysts used in the processes of this invention, appear to exhibit greater stabilities in the presence of halogen-containing vapor phase additive than catalysts prepared from, e.g., the chloride salt, under similar oxidative coupling conditions. In the most preferred aspects of the invention, the processes oxidatively couple methane to produce $C_2$'s.

In the oxidative coupling processes of this invention, lower alkane, e.g., 1 to 3 carbon atoms and reactive oxygen-containing material and halogen-containing vapor phase additive are fed to a reaction zone containing a catalytically effective amount of oxidative coupling catalyst, said oxidative coupling catalyst comprising a catalytically active amount of at least one of barium hydroxide, strontium hydroxide, barium carbonate and strontium carbonate on a support; the reaction zone is maintained with oxidative coupling conditions to convert at least a portion of the alkane to higher hydrocarbon and withdrawing an effluent containing higher hydrocarbon produced in the reaction zone from the reaction zone.

## Detailed Discussion of the Invention

In accordance with this invention, lower alkane is converted to higher hydrocarbon. The lower alkane preferably comprises at least one of methane, ethane and propane, and because of its abundance and the desire to convert it to higher hydrocarbons, methane is the most preferred component in the feed. The products of the conversion are higher hydrocarbons, especially alkanes and alkenes. Often, the desired conversion products are alkenes of two to four carbon atoms, especially ethylene and propylene. Because of its widespread use in commodity chemicals, product mixtures exhibiting a high selectivity to ethylene are typically preferred. The reaction is conducted in the presence of a reactive oxygen-containing material (oxidizing material) which for the purposes herein means atomic or molecular oxygen or a compound or chemical complex that contains an oxygen atom available for the oxidative coupling.

The hydrocarbon conversion process is by a cofeed, or simultaneous process, in which both the oxidizing material and the alkane-containing feed are provided at the same time to the reaction zone.

In a cofeed mode, the oxidizing material and alkane may be introduced by one or more separate streams or, most commonly, in a premixed stream. Generally, the mole ratio of alkane to active oxygen atom of the oxidizing material (an active oxygen atom is an oxygen atom that is available for oxidation) is at least about 1:2, say, about 1:2 to 50:1, preferably 1:1 to 20:1. The alkane typically comprises at least about 2 volume percent, e.g., up to about 95, say, 5 to 90, volume percent of the total gases fed to the reaction zone. Frequently, the feed streams are diluted with essentially inert gases such as those discussed above as helium, argon, steam, and carbon dioxide. When diluted, the diluent usually provides between about 5 to 95 volume percent of the feed streams.

The oxidizing material may be any suitable oxygen-bearing material which, under the conditions in the reaction zone, yields an active oxygen atom for the oxidative coupling. While not wishing to be limited to theory, the oxygen atom may be provided as reactive in a gaseous zone and/or may be provided on a catalyst surface as, for instance, reacted, absorbed or adsorbed form. Convenient oxidizing materials are normally gaseous such as molecular oxygen, (e.g., as oxygen, enriched air or air), ozone and gases which yield oxygen such as $N_2O$. Materials that are liquid or solid at ambient conditions may also be used provided that they can be facilely introduced into the reaction zone.

The reaction proceeds at elevated temperatures. Generally, a minimum temperature must be achieved before significant high hydrocarbon production occurs. If the temperature is too high, an undue amount of the hydrocarbon is consumed in oxidation or degradation reactions. Often, the temperature is in the range of about 500° to 1000°C., e.g., about 600° to 800°C. The reactants are usually preheated prior to their introduction into the reaction zone; for instance, to within about 200°C, preferably about 100°C of the temperature in the reaction zone.

The pressure in the reaction zone may vary widely from less than atmospheric to 100 atmospheres absolute or more often in the range of about 1 to 100, say, 1 to 50, atmospheres absolute.

In general, the reactions proceed rapidly and, hence, the reactants may reside in the reaction zone under reaction conditions for a relatively short period of time, e.g., less than about 20 seconds, often less than about 10 seconds. Frequently, the residence time is about 0.001 to 5, say, 0.1 to 3, seconds. The gas hourly space velocity based on the total gases fed to the reaction zone to the volume of the reaction zone is often about 50 to 50,000, preferably, 500 to 15000, reciprocal hours. Since alkane conversion reactions do not require the presence of catalyst to proceed, the overall volume of the vessel in which the reaction takes place may be substantially larger than that of the reaction zone containing catalyst. Even so, the volume of the reaction zone is frequently calculated as the volume of the vessel filled with catalyst.

The reaction may be conducted in any suitable reactor capable of providing the reaction temperatures. The reaction may be conducted in a single or in a series of sequential and/or parallel reactors. The catalyst bed may be of any suitable type, including, but not limited to, fixed, fluid, riser, falling, ebulating, and moving bed.

The catalyst size and configuration may vary depending upon the reactor type. For fluid, ebulating and riser reactors, the catalyst is typically between about 30 and 300 microns in major dimension. In fixed bed reactors, the catalyst may be in any suitable configuration including spheres, pellets, cylinders, monoliths, etc., and the size and shape may be influenced by pressure drop considerations for the gases passing through the bed. Often, the catalyst is at least about 0.2 centimeter, say, about 0.5 to 2 centimeters, in major dimension. Monolithic catalysts, can be sized to fit the reactor volume.

As states above, the catalysts of this invention contain a catalytically effective amount of at least one of barium hydroxide, strontium hydroxide, barium carbonate and strontium carbonate to effect the oxidative coupling reaction. The exact species of barium and/or strontium which is catalytically effective and the precise mechanism by which the barium catalyst performs the catalytic function is not known. The barium and strontium carbonates undergo a period of activation. Hence, it is likely that a catalytically active species is other than the carbonate. Similarly, the hydroxides undergo a period of activation and provide substantially virtually the same catalyst performance, all other things being equal, as is provided by catalyst prepared using the compounding carbonate. However, since the catalysts used in the processes of this invention retain higher activities under given conditions than those prepared from, say, barium chloride or

5

strontium chloride, the composite catalyst prepared from the carbonate salt and operated under halogen-containing environment, is believed to be unique. Barium oxide and strontium oxide are highly hygroscopic, and when in the unhydrated form, are generally more difficult to fabricate into useful catalyst configurations than the corresponding carbonates and hydroxides. Moreover, frequently commercial supplies of barium oxide and strontium oxide contain impurities which may be deleterious to catalytic performance. Hence, for purposes herein, the oxides are encompassed within the term hydroxides, which includes hydrated oxides.

The catalysts are supported on a catalytically-acceptable support. Unsupported barium or strontium do not appear to exhibit the yields to higher hydrocarbons and stabilities of the catalysts used in the processes of the invention. The support may be substantially inert under oxidative coupling conditions, such as low surface area, high purity alpha alumina, or may, by itself, have some activity, e.g., alpha aluminas, silica, titania, zirconia, calcium carbonate, calcium oxide, magnesium oxide, spinels, aluminosilicates, etc. The support is preferably porous and has a surface area (as determined by nitrogen by the B.E.T. method J. Am. Chem. Soc., vol. 60, 309 (1938)) of at least about 0.1, preferably at least about 0.2, say, 0.2 to 60 or 100 or more, square meters per gram.

The amount of barium hydroxide, strontium hydroxide, barium carbonate and strontium carbonate on the support may vary widely, e.g., from about 0.5 to 30, preferably from about 1 or 2 to 20, weight percent based on the total weight of the catalyst. The amount of barium hydroxide, strontium hydroxide, barium carbonate and strontium carbonate should be sufficient that when activated and under Standard Reference Conditions, as defined below, the yield of $C_2$'s is at least about 15 mole percent based on methane fed to the reaction zone. In some instances, the yield of $C_2$'s is at least about 20 mole percent under these conditions.

| Standard Reference Conditions | |
|---|---|
| Catalyst: | Ground to 30 to 90 mesh (U.S. Sieve Series) Approximately 1 to 10 grams. |
| Reaction Zone: | Quartz tube (at catalyst zone) 7 millimeters inside diameter, feed streams mixed immediately before reactor zone. |
| Temperature: | 750° C |
| Pressure: | 11 pounds per square inch gauge (176 kPa) |
| GHSV: | 800-1200 hr$^{-1}$ |
| Feed | |
| Methane: | 10 mole percent |
| Oxygen: | 5 mole percent |
| Nitrogen: | balance |
| Ethyl chloride: | to maximize yield (steady state) |
| Time of determination: | When stabilized between 50 and 100 hours of operation. |

The catalysts may contain one or more alkali and other alkaline earth metal components. If present, these components are generally in a mole ratio to total barium hydroxide, strontium hydroxide, barium carbonate and strontium carbonate of at least 0.01:1, say, about 0.1:1 to 10:1, and typically, 0.1:1 to 1:1. These components comprise the oxides, hydroxides, and/or other salts of one or more of sodium, lithium, potassium, rubidium, cesium, beryllium, magnesium and calcium and other salts of strontium and barium. When present, the salts may be halides $(X^-)$, oxyhalides $(OX^-)$, halites $(XO_2-)$, halates $(XO_3^-)$ and perhalates $(XO_4^-)$, wherein is one or more of chlorine, bromine and iodine; carbonates; sulfates; phosphates; nitrates; etc. Catalysts used in accordance with this invention often have an essential absence of alkali metal compounds, particularly alkali metal compounds which tend to vaporize, or be converted to compounds that tend to vaporize, under oxidative coupling reaction conditions.

The catalysts used in the processes of this invention may contain other adjuvants such as Group IIIA (including lanthanide series) components such as lanthanum oxide, Group IVA components (e.g., $TiO_2$, $ZrO_2$), Group VA components, Group VIA components and manganese. These other adjuvants may be present in amount of about 0.0001 to 10 or more weight percent based on the total weight of the catalyst. Silver and/or silver compound may also be present in an amount sufficient to enhance catalyst performance, e.g., 0.01 to 30, weight percent based on the total weight of the catalyst.

6

The catalysts may comprise at least one of cobalt, nickel, and rhodium in an amount up to about 2 weight percent based on the weight of the catalyst which amount is sufficient to provide enhanced oxidative coupling activity. Even though a relatively small amount of cobalt, nickel and/or rhodium are present in the catalyst, the processes are able to achieve substantially enhanced alkane conversion and/or selectivity to higher hydrocarbons at lower bulk reaction temperatures. Moreover, in preferred aspects of the invention, the cobalt and/or nickel and/or rhodium is provided in an amount sufficient to enhance the ethylene to ethane mole ratio in the reactor zone effluent as compared to a similar catalyst which does not contain the aforementioned component under the same conditions. The total amount of cobalt and/or nickel and/or rhodium compound is frequently at least about 0.01, e.g., about 0.01 to 1, say, about 0.2 to 0.8, weight percent (calculated as the metal) based on the total weight of the catalyst. the cobalt, nickel or rhodium may be initially provided on the catalyst in elemental or combined form (e.g., as a nitrate, oxide, hydroxide, carbonate, chloride, etc.). while not wishing to be limited to theory, it is believed that the metal should be maintained in at least a partially chemically combined form (e.g., as in one or more of oxide, carbonate, hydroxide, and halide, especially a chloride, bromide or iodide) during the process. See United States Patent Application Serial No. 409,375 filed on September 18, 1989, hereby incorporated by reference.

The supported catalysts may be prepared by any convenient technique. Techniques which have been proposed include coating the catalyst support with a slurry or paste of the ingredients or impregnating the support using a solution or suspension or complex of the ingredients (the impregnation may be simultaneous for all components or sequential). The impregnation may be by an incipient wetness technique or by immersion in the mother liquor or by evaporating solvent from a solution or suspension containing the support. The catalysts may be dried and, optionally, calcined.

A vapor phase halogen component is provided to the reaction zone during the process. It may be added intermittently or continuously. The halogen component may be provided as a solid, liquid or vapor when added. The halogen component may be halogen, e.g., chlorine, bromium or iodine, or a halogen-containing compound. The halogen-containing compounds (chlorine, bromine and iodine-containing compounds) may be inorganic or organic such as hydrogen halide, carbon tetrahalide, methylene halide, methyl dihalide, methyl trihalide, ethyl halide, ethyl dihalide, ethyl trihalide, ethyl tetrahalide, vinyl halide, sulfonyl chloride, phosphonyl chloride, etc. Often, the organic halides have from 1 to 3 halogen atoms and 1 to 3 carbon atoms. The amount of halogen component which can be added to the process, can vary; however, the amount added should be sufficient to provide the desired yield of higher hydrocarbon and lifetime of the catalyst. With either too little or too much halogen component addition, the catalyst performance will be adversely effected. Most frequently, if too little or too much halogen component has been added, good performance can be achieved by altering the rate of halogen component addition.

The amount of halogen component to be added for a given catalyst system will depend not only on the amount of barium and strontium hydroxides and carbonates on the catalyst, but also on the nature of the support and the presence of any adjuvants. For instance, optimum performance with a titania support may require more halogen component to achieve than that required for an alpha alumina support, all other catalyst and process factors being equal. Moreover, the optimum amount may change with the use of the catalyst.

Also, the type of halogen being added will influence the performance of the reaction system. In general, a process using a bromine compound as the halogen will provide a higher ratio of unsaturated to saturated hydrocarbons than a similar process using chlorine compound as the halogen. Within these guidelines, the amount of continuous vapor phase addition of the halogen component is often within the range of 0.1 to 5000, say, 1 to 1000, parts per million by volume based on the volume of feed to the reaction zone.

As stated above, the catalysts used in accordance with this invention activate upon initial use. The activation is often with respect to increasing methane conversion. The selectivity to heavier hydrocarbon is also frequently increased. Most preferably, the yield of alkane to heavier hydrocarbon is increased. In some instances, this yield increases by at least 50 percent upon activation. The activated catalyst produced by the processes of this invention and the processes of activating the catalyst are important aspects of this invention.

A volatilized metal component may be introduced intermittently or continuously into the reaction zone in an amount sufficient to reduce the rate of vapor phase alkane conversion reaction. Enhancements in selectivities to higher hydrocarbons may be obtained using the volatilized metal component additive, and the selectivity benefits provided by the catalysts better realized. See United States Patent Application Serial No. 409,361, filed on September 18, 1989, hereby incorporated by reference.

In practice, the amount of volatilized metal component to be added can be ascertained by monitoring the selectivity to higher hydrocarbons and adjusting the amount introduced. The amount of volatilized metal component introduced can vary widely and will depend, to some extent, on the nature of the volatilized

7

metal component. Often, the amount of volatilized metal component introduced is at least about 0.001 picogram, say, about 0.005 picogram to 10,000 or more milligrams per cubic meter of alkane in the feed (determined at standard temperature and pressure ("STP")).

It is not essential, and indeed in most instances it is not the case, that the volatilized metal component has a boiling point below the reaction temperature. Most convenient volatilized metal components have boiling points much higher than the reaction temperature under reaction conditions. However, the volatilized metal component should have a vapor pressure under the reaction conditions sufficient to provide the sought amount of volatilized metal component to achieve the sought reduction in vapor phase alkane conversion. Accordingly, the volatilized metal components are preferably molten or near to becoming molten (e.g, within 100°C) at the reaction temperature. The melting points of some volatilized metal components are set forth in Table I.

TABLE I

| VOLATILIZED COMPONENT | APPROXIMATE MELTING POINT (°C) |
|---|---|
| Barium chloride | 963 |
| Strontium chloride | 875 |
| Barium bromide | 847 |
| Sodium chloride | 801 |
| Calcium chloride | 782 |
| Potassium chloride | 770 |
| Sodium bromide | 758 |
| Barium iodide | 740 |
| Potassium bromide | 730 |
| Rubidium chloride | 718 |
| Potassium iodide | 686 |
| Sodium iodide | 653 |
| Cesium chloride | 645 |
| Strontium bromide | 643 |
| Cesium iodide | 612 |
| Lithium chloride | 605 |
| Barium hydroxide | 408 |
| Potassium hydroxide | 405 |
| Sodium hydroxide | 318 |
| Cesium hydroxide | 272 |

The preferred volatilized metal components are salts of Group IA and Group IIA metals. Salts such as nitrates, chromates, etc., may have explosive characteristics at the reaction temperatures. Thus, these salts and others which may adversely decompose or oxidize are generally avoided. The volatilized metal component, however, may be added in the form of an oxide, hydroxide, peroxide, superoxide or salt and be converted to another compound under the reaction conditions. In general, the preferred salts are halides, especially chlorides, bromides and iodides.

The introduction of the volatilized metal component into the reaction zone may be by any convenient means. Advantageously, the volatilized metal component is relatively uniformly distributed as it passes through the reaction zone. The introduction may be, for instance, by adding a stream of volatilized metal component in the vapor form to the reaction zone or to the feed stream. Since most volatilized metal components are not gases under the reaction conditions, the volatilized metal components must enter the vapor phase through sublimation or the effects of partial pressure over the metal component in the liquid state. Hence, it is often desirable to pass, at an elevated temperature (e.g. 400° to 1000°C, say, 500° to 850°C), all or a portion of the feed gas over the metal component to volatilize a desired amount of the metal component. Since oxidation reactions can occur at these elevated temperatures, frequently the volatilized metal component is contacted with either an alkane-containing stream having an essential absence of oxygen or a diluent gas or an oxygen-containing stream having an essential absence of alkane. The stream can be admixed with the remaining portion of the feed gases (for a continuous process).

In the event that the volatilized metal component solidifies, coalesces or is adsorbed or absorbed in the reaction zone, a build-up of the volatilized metal component may occur. In many instances, the build-up is not unduly deleterious; however, if it adversely affects the performance of the reaction, temporary cessation of the introduction of the volatilized metal component may be indicated.

The following examples are provided by way of illustration of the invention and are not in limitation thereof. All parts and percentages of solids are by weight and of liquids and gases are by volume unless otherwise noted or clear from the context.

Catalysts are made by one of two general procedures, Procedure A or Procedure B. The proper mesh of support is obtained from sieving and/or grinding and sieving supports prior to Procedures A and B. In Procedure A, supported catalysts are prepared using the incipient wetness technique. In this procedure, the amounts of components required to give the desired loadings are dissolved in a quantity of deionized, distilled water necessary to just fill the pores of the support. The solution is then added to the support particles. In some cases, if the dopants are not easily soluble, suspensions of the components are added to the support. The resulting material is dried in a vacuum oven at 130°C under a vacuum of 16-84 kPa for 1 to 50 hours (preferred 16 - 20 hours). Some of the dried catalysts are either tested without further treatment or first calcined in air at atmospheric pressure (usually at 800°C to 850°C). Metal or inorganic compound loadings are expressed as weight percent based on 100 weight percent catalyst.

In Procedure B, supported catalysts are prepared by adding the proper amount of component(s) to a mixture of water which is stirred with the support, while heating in a glass container on a hotplate for 2 to 3 hours (or until almost no water is left), to distribute the material in and on the support. If the dopants are not easily soluble, they are finely ground first. Deionized, distilled water is used (50 milliliters unless stated otherwise). The resulting material is dried in a vacuum oven at 130°C under a vacuum of 16-84 kPa for 1 to 50 hours (preferred 16 - 20 hours). Some of the dried catalysts are then calcined in air at atmospheric pressure (usually at 800 to 850°C). Metal or inorganic compound loadings are expressed as weight percent based on 100 weight percent catalyst.

The catalysts are evaluated using a reactor system containing two parallel reactors to which may be fed a variety of gases. The reactor system consists of a gas feed system, two tubular microreactors (a steam feed system to one of these), two Lindberg tube furnaces, two liquid collection systems, an on-line gas analyzer with a stream selector, and a separate liquids analyzer.

Two Lindberg Mini-Mite (TM), model 55035, tube furnaces (800 watts, 115 volts, 1100°C maximum operating temperature) are used for heating the tubular microreactors. The entire cylindrical heated chamber is comprised of two halves, making up the split-hinge furnace design. Each half is a composite unit of Moldatherm (TM), Lindberg's high temperature ceramic fiber insulation, and a helically coiled alloy heating element.

Quartz reactors are used. These include Reactor A, Reactor C, and Reactor D. Reactors are operated vertically.

Reactor A is constructed of 1.5 centimeters inside diameter quartz tubing (1.7 cm o. d.) with thermocouple wells of 0.3 cm i. d. quartz tubing (0.5 cm o. d.) positioned along the center of the reactors. The thermocouple well in Reactor A extends through the catalyst bed (into the preheater region) which allows the temperatures of the preheater, catalyst bed and post-catalyst region to be monitored. Reactor A is 56.5 centimeters long, and the thermocouple well extends 33.75 centimeters into the reactor. The bottom of the heated region of the reactor is 12.75 centimeters above the bottom of the reactor. The top of the heated region is 46.75 centimeters above the bottom of the reactor. The post-catalyst volume is packed with 20/40 or 14/30 mesh (U. S. Sieve Services) quartz chips in order to minimize the high temperature post-catalyst residence time of the products, and to hold the catalyst in place. The bottom end of the reactor is connected to 1.0 centimeter inside diameter (1.2 centimeters o. d.) quartz tubing at a right angle to the reactor.

The center of the catalyst bed is 18.5 centimeters above the bottom of the oven, or 31.25 centimeters above the bottom of the reactor. When the center of the reactor is heated to 802°C under typical flow rates of gases, the bottom of the heated portion of the reactor is about 645°C.

Reactor C is constructed of 0.9 centimeter inside diameter quartz tubing (1.1 centimeters o. d.). Reactor C is 56.5 centimeters long. The bottom of the heated region of the reactor is 12.75 centimeters above the bottom of the reactor. The top of the heated region is 46.75 centimeters above the bottom of the reactor. The post-catalyst volume is packed with 20/40 or 14/30 mesh (U. S. Sieve Series) quartz chips in order to minimize the high temperature post-catalyst residence time of the products, and to hold the catalyst in place. The bottom end of the reactor is connected to 1.0 centimeter inside diameter (1.2 centimeters o. d.) quartz tubing at a right angle to the reactor. The ends of the reactor are made of quartz "O"-ring joints (1.5 centimeters i. d. at the inlet and 0.9 cm i. d. at the outlet) which allow easy placement of the reactor into the system.

The center of the catalyst bed is 18.5 centimeters above the bottom of the oven, or 31.25 centimeters above the bottom of the reactor. A similar temperature profile as in Reactor A is exhibited by Reactor C.

The top 30.5 centimeters of Reactor D is constructed of 0.9 centimeter inside diameter quartz tubing (1.1 cm o. d.). This is connected to 26 centimeters of 3 millimeters inside diameter (5 mm 0. d. ) quartz tubing. Reactor C is 56.5 centimeters long. The bottom of the heated region of the reactor is 12.75 centimeters above the bottom of the reactor. The top of the heated region is 43.75 centimeters above the bottom of the reactor.

The post-catalyst volume of the wider part of the reactor is packed with 20/40 or 14/30 mesh quartz chips, quartz wool, or both quartz chips and quartz wool in order to minimize the high temperature post-catalyst residence time of the products, and to hold the catalyst in place. The bottom end of the reactor is connected to 1.0 centimeter inside diameter (1.2 cm o. d.) quartz tubing at a right angle to the reactor.

The center of the catalyst bed is 18.5 centimeters above the bottom of the oven, or 31.25 centimeters above the bottom of the reactor. Reactor D exhibits a similar temperature profile to that of Reactor A.

The catalyst bed is formed in Reactor D by providing quartz chips within the range of 14 to 40 mesh (U. S. Sieve Series) below and above the catalyst bed, which is separated from the quartz chips by quartz wool. The length of the catalyst bed along the reactor varies, depending on the weight of catalyst used, the density of the catalyst, and the type of reactor used. Most catalysts are within the range of 30 to 100 mesh (U. S. Sieve Series), with many from 30 to 60 mesh and many from 60 to 100 mesh.

In the general operating procedure, the reactors are filled with washed quartz chips above and below the catalyst bed. Those portions of Reactor D with tubing of 0.3 centimeter i. d. are not filled with quartz. Quartz wool is used to separate quartz chips from the catalyst bed and to hold quartz chips in the reactor. Undiluted catalysts of varying mesh sizes are used except in cases where the catalyst is only available as a fine powder. In these cases, it is diluted with quartz chips within the 14 to 40 mesh range.

Charged reactors are pressurized under nitrogen to about 176 kPa absolute (11 psig) then flushed with nitrogen during heating. Gas mixtures entering and exiting the reactor system during reactions are analyzed at intervals using gas chromatography. The reactor pressure is maintained at about 176 kPa throughout the reaction, and nitrogen is the diluent gas. The feed for most experiments contains $CH_4/O_2/N_2$ in a mole ratio of about 2/1/17. After experiments, the reactant flow is terminated and the reactor is flushed with nitrogen while cooling. The defined terms as follows are used in the Tables.

| Temperature (Temp) | Temperature of the catalyst bed (C). |
|---|---|
| Time | Time since reactant flow is started (minutes). |
| $CH_4$ C | Mole % of methane reacted. |
| Sel | Selectivity to ethane + ethylene expressed as a percent, based on methane reacted. |
| Yield | Mole % of methane converted to $C_2$'s, calculated as ($CH_4$ conversion) X ($C_2$ selectivity)/100. |
| = /- | Mole ratio of ethylene to ethane in the product stream. |
| $CO_2/CO$ | Mole ratio of carbon dioxide to carbon monoxide in the product stream. |
| GHSV | Gas hourly space velocity (reciprocal hours) at (STP) |
| $CH_4$ In | Mole % of methane introduced in the feed stream. |
| $O_2$ In | Mole % of oxygen introduced in the feed stream. |
| ECl | Ethyl chloride feed rate in parts per million by volume based on total feed. |
| EBr | Ethyl bromide feed rate in parts per million by volume based on total feed. |

### EXAMPLE 1

A 7 weight percent strontium carbonate (4.5 weight percent strontium) on alpha aluminum oxide catalyst is prepared by the general procedure A set forth above using 1.12 grams of strontium carbonate (from Johnson Matthey/AESAR Group, Seabrook, NH, Lot number S94313C, 99.999 weight percent) and 15.00 grams of alpha aluminum oxide (from Norton Company, Akron, OH, Sample number 8883118, Type SA 5451, B. E. T. surface area 0.27 square meter per gram). The catalyst is calcined at 850°C for 4 hours. The catalyst particle size is 60 to 100 mesh (U. 5. Sieve Series). The performance of the catalyst, using the equipment and procedures described above, with 5 grams of catalyst in Reactor D is presented in Table II.

**TABLE II**

| Temp °C | $CH_4$ C % | Sel % | Yield % | =/- | $CO_2/CO$ | $CH_4$ in Mole % | $O_2$ in Mole % | Time Min. | GHSV hr-1 | EC1 ppmv |
|---|---|---|---|---|---|---|---|---|---|---|
| 750 | 38.8 | 38.6 | 14.9 | 1.9 | 2.1 | 9.5 | 4.9 | 295 | 2400 | 5 |
| 750 | 38.6 | 39.1 | 15.0 | 1.9 | 2.2 | 9.5 | 4.9 | 385 | 2400 | 5 |
| 750 | 36.0 | 44.1 | 15.6 | 1.8 | 1.5 | 9.7 | 4.9 | 6100 | 2400 | 5 |
| 750 | 34.4 | 45.2 | 15.4 | 1.8 | 1.4 | 9.6 | 4.9 | 7180 | 2400 | 5 |
| 750 | 34.3 | 45.6 | 15.7 | 1.9 | 1.4 | 9.4 | 4.9 | 7450 | 2400 | 50 |
| 750 | 34.9 | 45.8 | 16.0 | 2.0 | 1.3 | 9.4 | 4.9 | 7540 | 2400 | 50 |
| 750 | 34.9 | 50.0 | 17.3 | 2.1 | 1.2 | 9.4 | 4.8 | 8980 | 2400 | 50 |
| 750 | 33.0 | 51.0 | 16.9 | 2.2 | 1.2 | 9.5 | 4.9 | 9745 | 2400 | 50 |
| 750 | 31.8 | 53.1 | 16.8 | 1.9 | 1.2 | 9.5 | 4.9 | 10005 | 2400 | 50 |
| 750 | 30.9 | 54.2 | 16.8 | 1.9 | 1.3 | 9.5 | 4.9 | 10080 | 2400 | 50 |
| 750 | 32.1 | 55.8 | 17.6 | 1.9 | 1.3 | 9.5 | 4.8 | 10285 | 2400 | 50 |
| 750 | 30.3 | 56.0 | 17.1 | 1.9 | 1.4 | 9.8 | 4.8 | 10375 | 2400 | 500 |
| 750 | 27.8 | 59.0 | 16.4 | 1.8 | 1.5 | 9.8 | 4.8 | 10465 | 2400 | 500 |
| 750 | 17.2 | 74.7 | 12.6 | 2.0 | 0.4 | 9.9 | 4.8 | 12625 | 2400 | 500 |
| 750 | 16.9 | 76.7 | 12.8 | 1.8 | 0.6 | 9.8 | 4.8 | 12795 | 2400 | 5 |
| 750 | 20.3 | 74.2 | 14.9 | 2.1 | 0.8 | 9.8 | 4.8 | 12890 | 2400 | 5 |
| 750 | 20.8 | 72.3 | 14.9 | 2.0 | 0.9 | 9.8 | 4.8 | 12950 | 2400 | 5 |
| 750 | 18.5 | 65.8 | 12.1 | 1.2 | 1.0 | 9.7 | 4.8 | 14060 | 2400 | 5 |
| 750 | 18.5 | 60.3 | 10.9 | 1.0 | 0.8 | 9.8 | 4.8 | 14600 | 2400 | 0 |

**TABLE II (continued)**

| Temp °C | CH₄ C % | Sel % | Yield % | =/- | CO₂/CO | CH₄ in Mole % | O₂ in Mole % | Time Min. | GHSV hr-1 | EC1 ppmv |
|---|---|---|---|---|---|---|---|---|---|---|
| 750 | 18.2 | 59.1 | 10.6 | 1.0 | 0.8 | 9.8 | 4.8 | 14735 | 2400 | 0 |
| 750 | 17.0 | 54.1 | 9.2 | 0.9 | 0.7 | 9.5 | 4.8 | 15500 | 2400 | 0 |
| 750 | 16.8 | 54.6 | 9.2 | 0.9 | 0.7 | 9.6 | 4.8 | 15745 | 2400 | 5 |
| 750 | 17.1 | 54.1 | 9.3 | 0.9 | 0.7 | 9.6 | 4.8 | 16045 | 2400 | 5 |
| 750 | 16.8 | 53.4 | 8.9 | 0.8 | 0.6 | 9.8 | 4.8 | 22700 | 2400 | 5 |

EXAMPLE 2 (comparative)

A 12 weight percent hydrated strontium chloride (4.5 weight percent strontium) on alpha aluminum oxide catalyst is prepared by the general procedure A set forth above using 2.9 grams of hydrated strontium chloride (6 waters of hydration, from Johnson Matthey/AESAR Group, Seabrook, NH, Lot number S94313C, 99.9965 weight percent) and 15.00 grams of alpha aluminum oxide (from Norton Company, Akron, OH, Sample number 8883118, Type SA 5451, B. E. T. surface area 0.27 square meter per gram). The catalyst is calcined at 850°C for 4 hours. The catalyst particle size is 60 to 100 mesh (U. 5. Sieve Series). The performance of the catalyst using the equipment and procedures described above is presented in Table III. Reactor D is used with 5 grams of catalyst.

12

TABLE III

| Temp °C | CH₄ C % | Sel % | Yield % | =/- | CO₂/CO | CH₄ in Mole % | O₂ in Mole % | Time Min. | GHSV hr-1 | ECI ppmv |
|---|---|---|---|---|---|---|---|---|---|---|
| 750 | 28.5 | 60.5 | 17.2 | 3.9 | 0.5 | 9.5 | 4.9 | 210 | 1200 | 5 |
| 750 | 29.6 | 62.3 | 18.2 | 4.0 | 0.6 | 9.5 | 4.9 | 340 | 1200 | 5 |
| 750 | 43.3 | 54.1 | 22.8 | 5.4 | 2.2 | 9.5 | 4.9 | 3265 | 1200 | 5 |
| 750 | 23.9 | 60.2 | 14.0 | 1.5 | 2.1 | 9.6 | 4.9 | 7225 | 1200 | 5 |
| 750 | 22.4 | 60.3 | 13.4 | 1.5 | 1.9 | 9.4 | 4.9 | 7495 | 1200 | 50 |
| 750 | 22.5 | 60.6 | 13.4 | 1.5 | 1.9 | 9.4 | 4.9 | 7585 | 1200 | 50 |
| 750 | 19.5 | 64.9 | 12.6 | 1.7 | 1.2 | 9.5 | 4.9 | 9790 | 1200 | 50 |
| 750 | 21.1 | 65.1 | 13.4 | 1.7 | 1.2 | 9.5 | 4.9 | 10040 | 1200 | 50 |
| 750 | 19.1 | 65.5 | 12.4 | 1.6 | 1.2 | 9.5 | 4.9 | 10120 | 1200 | 50 |
| 750 | 20.4 | 65.4 | 13.1 | 1.6 | 1.1 | 9.5 | 4.9 | 10230 | 1200 | 50 |
| 750 | 21.5 | 63.9 | 13.4 | 1.7 | 1.0 | 9.8 | 4.8 | 10310 | 1200 | 500 |
| 750 | 21.2 | 66.5 | 14.0 | 2.1 | 0.8 | 9.8 | 4.8 | 10420 | 1200 | 500 |
| 750 | 19.4 | 65.1 | 12.6 | 3.1 | 0.2 | 9.7 | 4.8 | 12750 | 1200 | 500 |
| 750 | 22.0 | 65.6 | 14.3 | 2.8 | 0.6 | 9.8 | 4.8 | 12850 | 1200 | 5 |
| 750 | 24.0 | 64.7 | 15.3 | 3.0 | 0.7 | 9.8 | 4.8 | 12925 | 1200 | 5 |
| 750 | 24.9 | 63.2 | 15.6 | 3.0 | 0.9 | 9.8 | 4.8 | 13040 | 1200 | 5 |
| 750 | 19.0 | 63.7 | 11.9 | 1.4 | 1.3 | 9.7 | 4.8 | 14105 | 1200 | 5 |
| 750 | 18.4 | 58.2 | 10.5 | 1.3 | 1.0 | 9.8 | 4.8 | 14545 | 1200 | 0 |
| 750 | 17.7 | 58.9 | 10.2 | 1.2 | 1.0 | 9.8 | 4.8 | 14660 | 1200 | 0 |
| 750 | 15.9 | 56.5 | 8.9 | 1.1 | 1.0 | 9.5 | 4.8 | 15425 | 1200 | 0 |
| 750 | 14.6 | 56.2 | 8.2 | 1.1 | 1.0 | 9.6 | 4.8 | 15785 | 1200 | 5 |
| 750 | 15.7 | 55.9 | 8.7 | 1.1 | 1.0 | 9.6 | 4.8 | 16085 | 1200 | 5 |
| 750 | 15.3 | 51.6 | 7.8 | 0.9 | 0.9 | 9.8 | 4.8 | 22745 | 1200 | 5 |

## EXAMPLE 3

A 29.87 weight percent barium carbonate (29.65 weight percent barium) on alpha aluminum oxide catalyst is prepared by the general procedure B set forth above using 2.556 grams of barium carbonate (from Johnson Matthey/AESAR Group, Seabrook, NH, Lot number S96252B, 99.997 weight percent) and 6 grams of alpha aluminum oxide (from Norton Company, Akron, OH, Sample number 8883118, Type SA 5451, B. E. T. surface area 0.27 square meters per gram) except 100 milliliters of water are used. The catalyst is calcined at 600°C for 14 hours and then 850°C for 4 hours. The catalyst particle size is 60 to 100 mesh (U. S. Sieve Series). The performance of the catalyst using the equipment and procedures described above is presented in Table IV. Reactor D is used with 5 grams of catalyst.

EP 0 418 972 A1

## TABLE IV

| Temp °C | CH$_4$ C % | Sel % | Yield % | =/- | CH$_4$ in Mole % | O$_2$ in Mole % | Time Min. | GHSV hr-1 | EC1 ppmv |
|---|---|---|---|---|---|---|---|---|---|
| 750 | 40.9 | 32.1 | 13.2 | 2.1 | 9.6 | 4.9 | 80 | 2133 | 10 |
| 750 | 42.0 | 34.0 | 14.2 | 2.4 | 9.6 | 4.9 | 205 | 2133 | 10 |
| 750 | 43.9 | 40.2 | 17.4 | 2.6 | 9.5 | 4.8 | 4075 | 2133 | 10 |
| 750 | 42.8 | 40.4 | 17.3 | 2.6 | 9.7 | 4.8 | 4165 | 2133 | 5 |
| 750 | 43.0 | 40.6 | 17.3 | 2.6 | 9.7 | 4.8 | 4255 | 2133 | 5 |
| 750 | 42.2 | 40.6 | 17.0 | 2.5 | 9.8 | 4.7 | 5575 | 2133 | 5 |
| 750 | 43.3 | 39.9 | 17.1 | 2.6 | 9.4 | 4.8 | 5665 | 2133 | 17 |
| 750 | 44.2 | 39.9 | 17.4 | 2.6 | 9.4 | 4.8 | 5755 | 2133 | 17 |
| 750 | 44.4 | 40.4 | 17.7 | 2.8 | 9.3 | 4.8 | 6520 | 2133 | 17 |
| 750 | 44.0 | 40.5 | 17.7 | 2.7 | 9.3 | 4.8 | 6700 | 2133 | 17 |
| 750 | 43.0 | 41.5 | 17.7 | 2.6 | 9.5 | 4.6 | 6835 | 2133 | 34 |
| 750 | 42.0 | 41.8 | 17.5 | 2.6 | 9.5 | 4.6 | 6920 | 2133 | 34 |
| 750 | 42.4 | 41.5 | 17.4 | 2.6 | 9.5 | 4.6 | 7000 | 2133 | 17 |
| 750 | 42.4 | 41.2 | 17.2 | 2.5 | 9.7 | 4.8 | 7190 | 2133 | 17 |
| 750 | 43.2 | 41.8 | 17.8 | 2.7 | 9.7 | 4.8 | 8210 | 2133 | 17 |
| 750 | 9.8 | 76.4 | 7.5 | 1.2 | 81.1 | 4.4 | 8300 | 1667 | 17 |
| 750 | 9.8 | 76.1 | 7.5 | 1.3 | 81.1 | 4.4 | 8370 | 1667 | 17 |

## TABLE IV (continued)

| Temp °C | CH₄ C % | Sel % | Yield % | =/- | CH₄ in Mole % | O₂ in Mole % | Time Min. | GHSV hr-1 | EC1 ppmv |
|---|---|---|---|---|---|---|---|---|---|
| 725 | 9.2 | 73.2 | 6.7 | 1.2 | 81.1 | 4.4 | 8485 | 1667 | 17 |
| 725 | 9.1 | 74.1 | 6.7 | 1.2 | 81.1 | 4.4 | 8515 | 1667 | 17 |
| 710 | 8.4 | 72.7 | 6.1 | 1.0 | 81.1 | 4.4 | 8615 | 1667 | 17 |
| 710 | 8.3 | 72.6 | 6.0 | 1.0 | 81.3 | 4.4 | 9840 | 1667 | 17 |
| 650 | 1.9 | 53.8 | 1.0 | 0.2 | 81.3 | 4.4 | 9975 | 1667 | 17 |
| 670 | 3.3 | 62.1 | 2.1 | 0.3 | 81.3 | 4.4 | 10040 | 1667 | 17 |
| 670 | 3.2 | 61.7 | 2.0 | 0.3 | 81.3 | 4.4 | 10095 | 1667 | 17 |
| 670 | 4.1 | 47.2 | 2.0 | 0.4 | 79.8 | 9.1 | 10165 | 1667 | 17 |
| 670 | 4.7 | 47.6 | 2.2 | 0.4 | 77.3 | 8.9 | 13900 | 1667 | 17 |
| 750 | 16.3 | 64.8 | 10.6 | 2.2 | 77.3 | 8.9 | 14035 | 1667 | 17 |
| 710 | 11.3 | 60.6 | 6.9 | 1.2 | 77.3 | 8.9 | 14115 | 1667 | 17 |

## TABLE IV (continued)

| Temp °C | CH$_4$ C % | Sel % | Yield % | =/- | CH$_4$ in Mole % | O$_2$ in Mole % | Time Min. | GHSV hr-1 | EC1 ppmv |
|---|---|---|---|---|---|---|---|---|---|
| 710 | 12.0 | 60.3 | 7.2 | 1.2 | 77.3 | 8.9 | 14210 | 1667 | 17 |
| 710 | 11.7 | 61.5 | 7.2 | 1.2 | 78.7 | 8.9 | 14420 | 1667 | 50 |
| 710 | 11.6 | 61.7 | 7.1 | 1.1 | 78.7 | 8.9 | 14530 | 1667 | 50 |
| 710 | 10.4 | 61.9 | 6.4 | 1.1 | 77.4 | 8.8 | 15835 | 1667 | 50 |
| 710 | 23.9 | 46.4 | 11.1 | 1.6 | 19.3 | 8.9 | 15925 | 1667 | 50 |
| 710 | 24.5 | 46.3 | 11.3 | 1.6 | 19.3 | 8.9 | 16015 | 1667 | 50 |
| 710 | 24.1 | 46.5 | 11.1 | 1.5 | 19.2 | 8.9 | 17095 | 1667 | 50 |
| 750 | 43.8 | 41.8 | 18.1 | 3.9 | 19.2 | 8.9 | 17170 | 1667 | 50 |
| 750 | 43.6 | 41.6 | 17.9 | 3.9 | 19.2 | 8.9 | 17200 | 1667 | 50 |
| 750 | 45.2 | 43.2 | 19.3 | 3.1 | 9.4 | 4.8 | 17275 | 1667 | 50 |
| 750 | 45.2 | 43.4 | 19.4 | 3.1 | 9.4 | 4.8 | 17330 | 1667 | 50 |
| 750 | 42.5 | 45.9 | 19.3 | 2.6 | 9.4 | 4.8 | 17455 | 2133 | 50 |
| 750 | 42.2 | 45.8 | 19.2 | 2.5 | 9.4 | 4.8 | 17635 | 2133 | 50 |
| 750 | 41.2 | 46.0 | 18.8 | 2.4 | 9.5 | 4.8 | 18220 | 2133 | 50 |
| 750 | 40.4 | 46.0 | 18.6 | 2.3 | 9.3 | 4.8 | 18355 | 2133 | 17 |
| 750 | 40.5 | 46.1 | 18.6 | 2.3 | 9.3 | 4.8 | 18445 | 2133 | 17 |
| 750 | 40.3 | 46.3 | 18.6 | 2.3 | 9.3 | 4.8 | 18535 | 2133 | 17 |

TABLE IV (continued)

| Temp °C | CH₄ C % | Sel % | Yield % | =/- | CH₄ in Mole % | O₂ in Mole % | Time Min. | GHSV hr⁻¹ | EC1 ppmv |
|---|---|---|---|---|---|---|---|---|---|
| 750 | 39.6 | 46.5 | 18.3 | 2.2 | 9.5 | 4.8 | 19660 | 2133 | 17 |
| 750 | 44.5 | 42.1 | 18.6 | 2.9 | 9.3 | 4.7 | 19795 | 1333 | 17 |
| 750 | 44.6 | 41.9 | 18.5 | 2.9 | 9.3 | 4.7 | 19885 | 1333 | 17 |
| 750 | 42.7 | 42.9 | 18.2 | 2.6 | 9.2 | 4.7 | 24115 | 1333 | 17 |
| 750 | 36.9 | 47.7 | 17.5 | 1.9 | 9.2 | 4.7 | 24205 | 2133 | 17 |
| 750 | 37.3 | 47.6 | 17.6 | 2.0 | 9.2 | 4.7 | 24295 | 2133 | 17 |

EXAMPLE 4

A 6.97 weight percent strontium carbonate (4.45 weight percent strontium) on alpha aluminum oxide catalyst is prepared by the general procedure B set forth above using 0.75 grams of strontium carbonate (from Johnson Matthey/AESAR Group, Seabrook, NH, Lot number S96275, 99.997 weight percent) and 10 grams of alpha aluminum oxide (from Norton Company, Akron, OH, Sample number 8883119, Type SA 5451, B. E. T. surface area 0.27 square meters per gram) except 40 milliliters of water are used. The catalyst is calcined at 850°C for 4 hours. The catalyst particle size is 30 to 60 mesh (U. S. Sieve Series). The performance of the catalyst using the equipment and procedures described above is presented in Table V. Reactor D is used with 2.5 grams of catalyst.

TABLE V

| Temp °C | CH₄ C % | Sel % | Yield % | =/- | CH₄ in Mole % | O₂ in Mole % | Time Min. | GHSV hr-l | ECI ppmv |
|---|---|---|---|---|---|---|---|---|---|
| 750 | 9.6 | 53.5 | 5.1 | 0.8 | 78.9 | 10.0 | 185 | 3300 | 10 |
| 750 | 11.2 | 55.0 | 6.1 | 0.9 | 78.9 | 10.0 | 275 | 3300 | 10 |
| 750 | 8.1 | 57.6 | 4.7 | 0.8 | 78.9 | 10.0 | 4405 | 3300 | 10 |
| 750 | 10.2 | 53.7 | 5.5 | 1.2 | 78.9 | 10.0 | 4495 | 2010 | 10 |
| 750 | 10.6 | 54.6 | 5.8 | 1.2 | 78.4 | 10.0 | 4585 | 2010 | 50 |
| 750 | 11.4 | 56.3 | 6.5 | 1.3 | 78.4 | 10.0 | 4675 | 2010 | 50 |
| 750 | 12.9 | 57.7 | 7.3 | 1.3 | 79.2 | 10.0 | 5710 | 2010 | 50 |
| 750 | 10.3 | 57.2 | 6.0 | 1.3 | 78.7 | 10.0 | 5800 | 2010 | 10 |
| 750 | 11.2 | 55.9 | 6.2 | 1.2 | 78.7 | 10.0 | 5890 | 2010 | 10 |
| 750 | 11.5 | 55.5 | 6.3 | 1.2 | 78.7 | 10.0 | 5970 | 2010 | 10 |

## EXAMPLE 5

A 3.6 weight percent strontium carbonate (2.22 weight percent strontium) on alpha aluminum oxide catalyst is prepared by the general procedure B set forth above using 0.5623 grams of strontium carbonate (from Johnson Matthey/AESAR Group, Seabrook, NH, Lot number S96275, 99.997 weight percent) and 15 grams of alpha aluminum oxide (from Norton Company, Akron, OH, Sample number 8883118, Type SA 5451, B. E. T. surface area 0.27 square meters per gram). The catalyst particle size is 60 to 100 mesh (U. S. Sieve Series). The performance of the catalyst using the equipment and procedures described above is presented in Table VI. Reactor D is used with 5 grams of catalyst.

EP 0 418 972 A1

## TABLE VI

| Temp °C | CH$_4$ C % | Sel % | Yield % | =/- | CH$_4$ in Mole % | O$_2$ in Mole % | Time Min. | GHSV hr-1 | EC1 ppmv |
|---|---|---|---|---|---|---|---|---|---|
| 750 | 35.8 | 29.6 | 10.6 | 2.5 | 9.9 | 4.9 | 30 | 783 | 0 |
| 750 | 34.2 | 27.4 | 9.4 | 2.3 | 9.9 | 4.9 | 105 | 783 | 0 |
| 750 | 30.4 | 27.9 | 8.5 | 1.9 | 9.9 | 4.9 | 195 | 1043 | 0 |
| 750 | 29.8 | 27.2 | 8.2 | 1.9 | 9.9 | 4.9 | 285 | 1043 | 0 |
| 750 | 25.7 | 21.5 | 5.5 | 1.4 | 10.0 | 4.9 | 3930 | 1043 | 0 |
| 750 | 19.3 | 23.0 | 4.4 | 0.9 | 10.0 | 4.9 | 4065 | 2087 | 0 |
| 750 | 19.0 | 23.0 | 4.4 | 0.9 | 10.0 | 4.9 | 4155 | 2087 | 0 |
| 750 | 21.2 | 29.2 | 6.2 | 1.2 | 10.0 | 5.0 | 4245 | 2087 | 30 |
| 750 | 27.4 | 39.8 | 11.0 | 1.7 | 10.0 | 5.0 | 4335 | 2087 | 30 |
| 750 | 35.0 | 47.9 | 16.8 | 2.5 | 9.8 | 4.8 | 5775 | 2087 | 30 |
| 775 | 42.7 | 44.6 | 19.0 | 3.4 | 9.8 | 4.8 | 5865 | 2087 | 30 |
| 775 | 42.0 | 44.8 | 18.9 | 3.4 | 9.8 | 4.8 | 5955 | 2087 | 30 |
| 775 | 40.7 | 47.1 | 19.1 | 3.3 | 9.9 | 5.1 | 6450 | 2087 | 30 |
| 775 | 38.0 | 49.3 | 18.6 | 3.1 | 9.9 | 4.9 | 7145 | 2087 | 30 |
| 800 | 44.0 | 45.2 | 19.8 | 4.4 | 9.9 | 4.9 | 7205 | 2087 | 30 |
| 750 | 27.8 | 54.5 | 15.0 | 2.0 | 9.9 | 4.9 | 7440 | 2087 | 30 |
| 750 | 27.4 | 55.3 | 15.0 | 2.0 | 9.9 | 4.9 | 7530 | 2087 | 30 |
| 750 | 25.0 | 57.2 | 14.2 | 1.9 | 10.1 | 5.0 | 8510 | 2087 | 30 |
| 700 | 11.7 | 50.7 | 5.9 | 0.7 | 10.1 | 5.0 | 8590 | 2087 | 30 |

## TABLE VI (continued)

| Temp °C | CH₄ C % | Sel % | Yield % | =/- | CH₄ in Mole % | O₂ in Mole % | Time Min. | GHSV hr-1 | EC1 ppmv |
|---|---|---|---|---|---|---|---|---|---|
| 750 | 25.3 | 58.1 | 14.5 | 1.9 | 10.1 | 5.0 | 8655 | 2087 | 30 |
| 750 | 28.1 | 56.2 | 15.6 | 2.3 | 10.1 | 5.0 | 8715 | 1696 | 30 |
| 750 | 23.9 | 58.7 | 14.0 | 1.9 | 9.9 | 4.9 | 8880 | 2087 | 40 |
| 750 | 23.5 | 60.0 | 14.0 | 1.9 | 9.9 | 4.9 | 8970 | 2087 | 40 |
| 750 | 22.0 | 61.2 | 13.4 | 1.8 | 10.1 | 4.7 | 9690 | 2087 | 40 |
| 770 | 28.6 | 59.4 | 16.9 | 2.6 | 10.0 | 5.0 | 9825 | 2087 | 40 |
| 770 | 28.5 | 59.1 | 16.7 | 2.5 | 10.0 | 5.0 | 9890 | 2087 | 40 |
| 770 | 28.6 | 57.8 | 16.4 | 2.4 | 10.0 | 5.0 | 10035 | 2087 | 40 |
| 760 | 24.7 | 59.9 | 14.7 | 2.0 | 10.0 | 5.0 | 10140 | 2087 | 40 |
| 760 | 24.0 | 60.6 | 14.5 | 2.0 | 10.0 | 5.0 | 10275 | 2087 | 40 |
| 760 | 24.3 | 60.6 | 14.6 | 2.0 | 10.0 | 5.0 | 10365 | 2087 | 40 |
| 760 | 15.4 | 67.2 | 10.3 | 1.3 | 9.9 | 4.7 | 14145 | 2087 | 40 |
| 760 | 14.7 | 66.4 | 9.8 | 1.2 | 10.0 | 4.8 | 14235 | 2087 | 15 |
| 760 | 15.3 | 63.8 | 9.8 | 1.2 | 10.0 | 4.8 | 14300 | 2087 | 15 |
| 760 | 15.1 | 62.3 | 9.5 | 1.2 | 9.8 | 4.8 | 14365 | 2087 | 0 |
| 760 | 15.7 | 60.6 | 9.6 | 1.2 | 9.8 | 4.8 | 14415 | 2087 | 0 |
| 760 | 16.4 | 58.2 | 9.7 | 1.2 | 9.8 | 4.8 | 14485 | 2087 | 0 |
| 760 | 19.5 | 53.3 | 10.4 | 1.2 | 9.8 | 4.6 | 14595 | 2087 | 5 |
| 760 | 19.3 | 54.2 | 10.5 | 1.2 | 9.8 | 4.6 | 14685 | 2087 | 5 |

EP 0 418 972 A1

## TABLE VI (continued)

| Temp °C | CH4 C % | Sel % | Yield % | =/- | CH4 in Mole % | O2 in Mole % | Time Min. | GHSV hr-1 | ECl ppmv |
|---|---|---|---|---|---|---|---|---|---|
| 760 | 17.9 | 55.5 | 9.9 | 1.2 | 10.0 | 4.8 | 15450 | 2087 | 5 |
| 760 | 18.8 | 55.5 | 10.3 | 1.2 | 10.0 | 4.7 | 15585 | 2087 | 0 |
| 760 | 19.8 | 54.2 | 10.6 | 1.2 | 10.0 | 4.7 | 15675 | 2087 | 0 |
| 760 | 21.8 | 49.6 | 10.7 | 1.3 | 10.0 | 4.7 | 15945 | 2087 | 0 |
| 760 | 23.0 | 36.9 | 8.4 | 1.2 | 9.9 | 4.8 | 17025 | 2087 | 0 |
| 760 | 22.6 | 35.9 | 8.1 | 1.2 | 10.0 | 4.7 | 17115 | 2087 | 200 |
| 760 | 20.1 | 45.5 | 9.2 | 1.4 | 10.0 | 4.7 | 17205 | 2087 | 200 |
| 760 | 14.8 | 69.8 | 10.4 | 1.7 | 10.0 | 4.7 | 17295 | 2087 | 200 |
| 760 | 14.2 | 70.8 | 10.1 | 1.7 | 10.0 | 4.7 | 17385 | 2087 | 200 |
| 760 | 11.9 | 73.8 | 8.8 | 1.4 | 9.8 | 4.7 | 17475 | 2087 | 10 |
| 760 | 10.8 | 68.9 | 7.4 | 1.0 | 9.8 | 4.7 | 17565 | 2087 | 10 |
| 760 | 10.4 | 62.5 | 6.5 | 0.8 | 10.0 | 4.8 | 17970 | 2087 | 10 |

## EXAMPLE 6

A 4.77 weight percent barium carbonate (3.49 weight percent barium) on alpha aluminum oxide catalyst is prepared by the general procedure B set forth above using 0.752 grams of barium carbonate (from Johnson Matthey/AESAR Group, Seabrook, NH, Lot number S96252B, 99.997 weight percent) and 15 grams of alpha aluminum oxide (from Norton Company, Akron, OH, Sample number 8883118, Type SA 5451, B. E. T. surface area 0.27 square meters per gram). The catalyst is calcined at 850°C for 4 hours. The catalyst particle size is 60 to 100 mesh (U. S. Sieve Series). The performance of the catalyst using the equipment and procedures described above is presented in Table VII. Reactor D is used with 5 grams of catalyst.

| Temp | CH₄ C | Sel % | Yield % | =/- | CH₄ in | O₂ in | Time | GHSV | ECI |
|---|---|---|---|---|---|---|---|---|---|
| C | % | | | | Mole % | Mole % | Min. | hr-l | ppmv |
| 750 | 32.8 | 37.4 | 12.3 | 1.8 | 10.2 | 5.2 | 70 | 2233 | 5 |
| 750 | 36.4 | 40.4 | 14.7 | 2.1 | 10.2 | 5.2 | 190 | 2233 | 5 |
| 750 | 43.9 | 44.3 | 19.2 | 3.5 | 10.2 | 5.2 | 640 | 2233 | 5 |
| 750 | 40.0 | 39.6 | 15.7 | 2.8 | 10.5 | 5.1 | 4240 | 2233 | 5 |
| 750 | 40.9 | 39.2 | 16.1 | 3.2 | 9.9 | 5.2 | 4330 | 2233 | 10 |
| 750 | 41.3 | 39.4 | 16.2 | 3.4 | 9.9 | 5.2 | 4420 | 2233 | 10 |
| 750 | 41.3 | 39.5 | 16.3 | 3.4 | 9.9 | 5.2 | 4555 | 2233 | 10 |
| 750 | 40.9 | 39.4 | 16.2 | 3.5 | 9.8 | 5.1 | 5545 | 2233 | 10 |
| 750 | 42.3 | 39.4 | 16.5 | 4.1 | 10.0 | 5.1 | 5680 | 2233 | 17 |
| 750 | 42.0 | 39.8 | 16.6 | 4.1 | 10.0 | 5.1 | 5770 | 2233 | 17 |
| 750 | 42.0 | 39.8 | 16.6 | 4.1 | 10.0 | 5.1 | 6040 | 2233 | 17 |
| 750 | 37.9 | 39.3 | 14.9 | 2.5 | 10.2 | 5.1 | 6175 | 2233 | 1 |
| 750 | 37.0 | 38.3 | 14.2 | 2.1 | 10.2 | 5.1 | 6400 | 2233 | 1 |

## EXAMPLE 7

A 5 weight percent barium carbonate (3.5 weight percent barium) on alpha aluminum oxide catalyst is prepared by the general procedure B set forth above using 0.75 gram of barium carbonate (from Johnson Matthey/AESAR Group, Seabrook, NH, Lot number 5962529, 99.997 weight percent) and 15 grams of alpha aluminum oxide (from Norton Company, Akron, OH, Sample number 8883118, Type SA 5451, 9. E. T. surface area 0.27 square meter per gram). The catalyst particle size is 60 to 100 mesh (U. S. Sieve Series). The performance of the catalyst using the equipment described above is presented in Table VIII. Reactor D is used with 5.0 grams of catalyst. This example shows the $C_2$ selectivity, $C_2$ yield, ethylene to ethane ratio, and methane conversion to increase when ethyl chloride is added to the catalyst. It may be seen that catalyst activation requires over 530 minutes after ethyl chloride is added to the gas feed under these conditions, and that changes in level of chloride have a significant effect on the catalyst performance. At 14725 minutes, it can be seen that catalyst performance is good, with a $C_2$ yield above 20%. In spite of changes in temperature and ethyl chloride level, stable performance of the catalyst is observed for over 4000 minutes after catalyst activation.

22

## TABLE VIII

| Temp °C | CH$_4$ C % | Sel % | Yield % | =/- | CH$_4$ in Mole % | O$_2$ in Mole % | Time Min. | GHSV hr-1 | EC1 ppmv |
|---|---|---|---|---|---|---|---|---|---|
| 750 | 38.4 | 33.1 | 12.8 | 2.0 | 9.9 | 4.9 | 60 | 783 | 0 |
| 750 | 37.7 | 31.4 | 11.9 | 1.8 | 9.9 | 4.9 | 140 | 783 | 0 |
| 750 | 36.7 | 31.1 | 11.5 | 1.8 | 9.9 | 4.9 | 240 | 1043 | 0 |
| 750 | 36.4 | 29.9 | 11.0 | 1.7 | 9.9 | 4.9 | 330 | 1043 | 0 |
| 750 | 34.9 | 25.0 | 8.8 | 1.6 | 10.0 | 4.9 | 3975 | 1043 | 0 |
| 750 | 34.1 | 27.2 | 9.3 | 1.4 | 10.0 | 4.9 | 4110 | 2087 | 0 |
| 750 | 34.3 | 26.8 | 9.2 | 1.4 | 10.0 | 4.9 | 4200 | 2087 | 0 |
| 750 | 35.8 | 30.0 | 10.9 | 1.8 | 10.0 | 5.0 | 4290 | 2087 | 30 |
| 750 | 38.0 | 33.9 | 13.0 | 2.1 | 10.0 | 5.0 | 4380 | 2087 | 30 |
| 750 | 39.9 | 48.3 | 19.3 | 2.2 | 9.8 | 4.8 | 5820 | 2087 | 30 |
| 760 | 43.3 | 46.8 | 20.2 | 2.6 | 9.8 | 4.8 | 5895 | 2087 | 30 |
| 760 | 42.8 | 47.1 | 20.2 | 2.5 | 9.8 | 4.8 | 6000 | 2087 | 30 |
| 760 | 42.4 | 47.5 | 20.2 | 2.5 | 9.8 | 4.8 | 6090 | 2087 | 30 |
| 760 | 42.3 | 48.1 | 20.3 | 2.5 | 9.8 | 5.0 | 6225 | 2087 | 30 |
| 760 | 41.7 | 48.7 | 20.3 | 2.4 | 9.8 | 5.0 | 6315 | 2087 | 30 |
| 760 | 41.3 | 49.5 | 20.4 | 2.4 | 9.9 | 5.1 | 6495 | 2087 | 30 |
| 760 | 40.6 | 50.2 | 20.4 | 2.4 | 9.9 | 5.1 | 6675 | 2087 | 30 |

EP 0 418 972 A1

## TABLE VIII (continued)

| Temp °C | CH4 C % | Sel % | Yield % | =/- | CH4 in Mole % | O2 in Mole % | Time Min. | GHSV hr-1 | EC1 ppmv |
|---|---|---|---|---|---|---|---|---|---|
| 760 | 39.6 | 51.7 | 20.5 | 2.3 | 9.9 | 5.1 | 6855 | 2087 | 30 |
| 775 | 45.2 | 49.5 | 22.2 | 3.2 | 9.9 | 4.9 | 6975 | 2087 | 30 |
| 775 | 45.3 | 49.6 | 22.3 | 3.2 | 9.9 | 4.9 | 7005 | 2087 | 30 |
| 775 | 45.3 | 49.7 | 22.4 | 3.3 | 9.9 | 4.9 | 7050 | 2087 | 30 |
| 780 | 46.8 | 49.6 | 23.1 | 3.8 | 9.9 | 4.9 | 7095 | 2087 | 30 |
| 780 | 46.8 | 50.0 | 23.3 | 3.9 | 9.9 | 4.9 | 7175 | 2087 | 30 |
| 785 | 48.2 | 50.0 | 24.0 | 4.8 | 9.9 | 4.9 | 7235 | 2087 | 30 |
| 785 | 47.8 | 50.2 | 24.0 | 4.9 | 9.9 | 4.9 | 7265 | 2087 | 30 |
| 790 | 48.9 | 50.4 | 24.6 | 5.8 | 9.9 | 4.9 | 7300 | 2087 | 30 |
| 790 | 49.0 | 50.5 | 24.7 | 6.0 | 9.9 | 4.9 | 7345 | 2087 | 30 |
| 800 | 50.4 | 52.4 | 26.0 | 7.2 | 9.9 | 4.9 | 7395 | 2087 | 30 |
| 800 | 50.0 | 53.2 | 26.1 | 6.8 | 9.9 | 4.9 | 7485 | 2087 | 30 |
| 800 | 47.5 | 50.8 | 23.9 | 5.5 | 10.1 | 5.0 | 8420 | 2087 | 30 |
| 790 | 46.4 | 51.6 | 23.7 | 4.9 | 10.1 | 5.0 | 8455 | 2087 | 30 |
| 790 | 46.3 | 51.8 | 23.8 | 4.9 | 10.1 | 5.0 | 8485 | 2087 | 30 |
| 790 | 46.4 | 51.6 | 23.5 | 4.8 | 10.1 | 5.0 | 8745 | 2087 | 30 |
| 790 | 45.5 | 52.4 | 23.6 | 4.8 | 9.9 | 4.9 | 8790 | 2087 | 40 |

## TABLE VIII (continued)

| Temp °C | CH$_4$ C % | Sel % | Yield % | =/- | CH$_4$ in Mole % | O$_2$ in Mole % | Time Min. | GHSV hr-1 | EC1 ppmv |
|---------|------------|-------|---------|-----|------------------|-----------------|-----------|-----------|----------|
| 790 | 45.6 | 52.2 | 23.6 | 4.9 | 9.9 | 4.9 | 8820 | 2087 | 40 |
| 790 | 44.9 | 52.9 | 23.6 | 4.9 | 9.9 | 4.9 | 8925 | 2087 | 40 |
| 790 | 42.9 | 54.1 | 23.0 | 4.6 | 10.1 | 4.7 | 9735 | 2087 | 40 |
| 800 | 46.1 | 51.8 | 23.5 | 5.1 | 10.0 | 5.0 | 9860 | 2087 | 40 |
| 800 | 46.0 | 51.4 | 23.4 | 5.1 | 10.0 | 5.0 | 9925 | 2087 | 40 |
| 780 | 38.5 | 57.3 | 21.9 | 3.5 | 10.0 | 5.0 | 9960 | 2087 | 40 |
| 760 | 25.4 | 66.9 | 16.8 | 2.0 | 10.0 | 5.0 | 10005 | 2087 | 40 |
| 760 | 23.7 | 67.7 | 15.9 | 2.0 | 10.0 | 5.0 | 10065 | 2087 | 40 |
| 740 | 14.5 | 70.1 | 10.1 | 1.1 | 10.0 | 5.0 | 10095 | 2087 | 40 |
| 740 | 13.3 | 71.0 | 9.4 | 1.0 | 10.0 | 5.0 | 10180 | 2087 | 40 |
| 790 | 41.2 | 56.3 | 22.9 | 4.9 | 10.0 | 5.0 | 10215 | 2087 | 40 |
| 790 | 42.0 | 55.6 | 23.0 | 4.8 | 10.0 | 5.0 | 10245 | 2087 | 40 |
| 790 | 31.8 | 60.1 | 19.0 | 3.2 | 9.9 | 4.7 | 14175 | 2087 | 40 |
| 790 | 31.4 | 59.9 | 18.8 | 3.2 | 10.0 | 4.8 | 14200 | 2087 | 15 |
| 790 | 32.6 | 58.7 | 19.1 | 3.0 | 10.0 | 4.8 | 14265 | 2087 | 15 |
| 790 | 33.7 | 58.1 | 19.5 | 3.1 | 10.0 | 4.8 | 14330 | 2087 | 15 |
| 790 | 34.4 | 57.0 | 19.7 | 3.1 | 9.8 | 4.8 | 14385 | 2087 | 0 |

## TABLE VIII (continued)

| Temp °C | CH₄ C % | Sel % | Yield % | */- | CH₄ in Mole % | O₂ in Mole % | Time Min. | GHSV hr-1 | ECl ppmv |
|---|---|---|---|---|---|---|---|---|---|
| 790 | 36.1 | 55.7 | 20.2 | 3.2 | 9.8 | 4.8 | 14450 | 2087 | 0 |
| 790 | 37.1 | 54.5 | 20.5 | 3.3 | 9.8 | 4.8 | 14545 | 2087 | 0 |
| 790 | 37.3 | 53.5 | 20.3 | 3.2 | 9.8 | 4.8 | 14635 | 2087 | 5 |
| 790 | 37.9 | 53.8 | 20.3 | 3.1 | 9.8 | 4.6 | 14725 | 2087 | 5 |
| 790 | 35.1 | 54.0 | 18.9 | 3.1 | 10.0 | 4.8 | 15310 | 2087 | 5 |
| 790 | 38.1 | 52.1 | 19.6 | 3.4 | 10.0 | 4.7 | 15445 | 2087 | 0 |
| 790 | 38.9 | 50.7 | 19.5 | 3.5 | 10.0 | 4.7 | 15535 | 2087 | 0 |
| 790 | 36.0 | 32.8 | 11.8 | 2.2 | 9.9 | 4.8 | 16885 | 2087 | 0 |
| 790 | 32.3 | 39.6 | 12.9 | 2.3 | 10.0 | 4.7 | 16975 | 2087 | 200 |
| 790 | 27.2 | 63.1 | 17.1 | 3.3 | 10.0 | 4.7 | 17065 | 2087 | 200 |
| 790 | 18.3 | 67.1 | 12.2 | 2.5 | 10.0 | 4.7 | 17240 | 2087 | 200 |
| 790 | 24.8 | 64.2 | 15.7 | 3.2 | 9.8 | 4.7 | 17335 | 2087 | 10 |
| 790 | 30.3 | 60.1 | 18.0 | 3.6 | 9.8 | 4.7 | 17425 | 2087 | 10 |
| 790 | 23.0 | 56.7 | 13.0 | 2.2 | 10.0 | 4.8 | 18190 | 2087 | 10 |

EXAMPLE 8 (comparative)

A 1.4 weight percent barium chloride (0.9 weight percent barium) on alpha aluminum oxide catalyst is prepared by the general procedure A set forth above using 0.14 gram of barium chloride (from Johnson Matthey/AESAR Group, Seabrook, NH, Lot number S94730, 99.9999 weight percent) and 10 grams of alpha aluminum oxide (from Norton Company, Akron, OH, Sample number 83111, Type SA 5551, B. E. T. surface area 0.25 square meter per gram). The catalyst particle size is 60 to 100 mesh (U. S. Sieve Series). The performance of the catalyst using the equipment described above is presented in Table IX. Reactor A is used with 5.0 grams of catalyst.

EP 0 418 972 A1

## TABLE IX

| Temp °C | CH$_4$ C % | Sel % | Yield % | =/- | CO$_2$/CO | CH$_4$ in Mole % | O$_2$ in Mole % | Time Min. | GHSV hr-1 | EC1 ppmv |
|---|---|---|---|---|---|---|---|---|---|---|
| 750 | 40.1 | 45.0 | 18.4 | 12.9 | 0.2 | 10.4 | 5.3 | 75 | 1143 | 500 |
| 750 | 39.6 | 41.4 | 16.6 | 13.8 | 0.2 | 10.4 | 5.3 | 165 | 857 | 500 |
| 750 | 38.4 | 41.6 | 16.1 | 14.0 | 0.2 | 10.4 | 5.3 | 255 | 857 | 500 |
| 750 | 37.5 | 41.6 | 15.8 | 13.9 | 0.2 | 10.4 | 5.3 | 345 | 857 | 500 |
| 750 | 35.1 | 43.9 | 15.6 | 10.7 | 0.2 | 10.5 | 5.1 | 1200 | 857 | 500 |
| 750 | 39.9 | 36.6 | 14.9 | 14.5 | 0.2 | 10.5 | 5.1 | 1245 | 571 | 500 |
| 750 | 40.0 | 36.6 | 14.9 | 14.4 | 0.2 | 10.5 | 5.1 | 1290 | 571 | 500 |
| 750 | 38.0 | 41.6 | 16.0 | 12.5 | 0.2 | 10.5 | 5.1 | 1335 | 571 | 500 |
| 750 | 38.0 | 41.7 | 16.1 | 12.4 | 0.2 | 10.5 | 5.1 | 1380 | 571 | 500 |
| 750 | 27.3 | 53.7 | 14.7 | 6.5 | 0.1 | 10.5 | 5.1 | 1425 | 1429 | 500 |
| 750 | 19.8 | 62.3 | 12.4 | 3.8 | 0.1 | 10.5 | 5.1 | 1470 | 2143 | 500 |
| 750 | 28.3 | 54.5 | 15.4 | 6.7 | 0.1 | 10.5 | 5.1 | 1515 | 1143 | 500 |
| 750 | 36.4 | 49.2 | 18.3 | 10.0 | 0.1 | 7.0 | 4.9 | 1605 | 1143 | 500 |
| 750 | 41.7 | 44.1 | 18.8 | 13.0 | 0.1 | 7.0 | 4.9 | 1650 | 857 | 500 |
| 750 | 46.0 | 40.7 | 19.4 | 16.2 | 0.1 | 5.7 | 4.9 | 1740 | 857 | 500 |
| 750 | 38.2 | 47.3 | 18.6 | 10.5 | 0.1 | 5.8 | 5.0 | 2640 | 1143 | 500 |
| 750 | 38.1 | 47.7 | 18.7 | 10.5 | 0.1 | 5.8 | 5.0 | 2685 | 1143 | 500 |
| 775 | 52.9 | 35.3 | 19.7 | 20.0 | 0.2 | 5.8 | 5.0 | 2730 | 1143 | 500 |
| 775 | 47.0 | 41.6 | 20.5 | 14.3 | 0.1 | 5.8 | 5.0 | 2865 | 1714 | 500 |

**TABLE IX (Continued)**

| Temp °C | CH₄ C % | Sel % | Yield % | =/- | CO₂/CO | CH₄ in Mole % | O₂ in Mole % | Time Min. | GHSV hr-1 | ECl ppmv |
|---|---|---|---|---|---|---|---|---|---|---|
| 750 | 35.1 | 50.1 | 18.2 | 9.0 | 0.1 | 5.8 | 5.0 | 2905 | 1429 | 500 |
| 775 | 40.0 | 47.8 | 19.9 | 10.5 | 0.1 | 5.8 | 5.0 | 2950 | 2286 | 500 |
| 775 | 39.6 | 48.0 | 19.8 | 10.4 | 0.1 | 5.8 | 5.0 | 2995 | 2286 | 500 |
| 775 | 41.6 | 47.5 | 20.2 | 6.3 | 0.4 | 5.5 | 5.0 | 3080 | 2286 | 100 |
| 775 | 41.9 | 46.9 | 20.1 | 6.3 | 0.5 | 5.5 | 5.0 | 3175 | 2286 | 100 |
| 775 | 42.0 | 47.6 | 20.0 | 6.0 | 0.5 | 5.6 | 5.0 | 3940 | 2286 | 100 |
| 800 | 60.2 | 33.4 | 20.8 | 13.3 | 0.4 | 5.6 | 4.9 | 4075 | 2286 | 100 |
| 750 | 23.8 | 59.3 | 14.0 | 2.6 | 0.5 | 5.6 | 4.9 | 4120 | 2286 | 100 |
| 750 | 37.9 | 47.1 | 17.9 | 5.4 | 0.5 | 5.6 | 4.9 | 4210 | 1143 | 100 |
| 725 | 14.9 | 62.0 | 9.2 | 1.6 | 0.4 | 5.6 | 4.9 | 4250 | 2286 | 100 |
| 725 | 36.3 | 42.6 | 15.5 | 5.4 | 0.5 | 5.6 | 4.9 | 4305 | 571 | 100 |
| 750 | 48.0 | 35.8 | 17.4 | 8.2 | 0.5 | 5.6 | 4.9 | 4350 | 857 | 100 |
| 750 | 35.6 | 42.9 | 15.3 | 5.7 | 0.5 | 9.9 | 5.3 | 4485 | 857 | 100 |

EXAMPLE 9 (Comparative)

A 5 weight percent barium chloride (3.5 weight percent barium) on alpha aluminum oxide catalyst is prepared by the general procedure A set forth above using 0.265 gram of barium chloride (from Johnson Matthey/AESAR Group, Seabrook, NH, Lot number S94730, 99.9999 weight percent) and 5 grams of alpha aluminum oxide (from Norton Company, Akron, OH, Sample number S94730, Type SA 5502, B. E. T. surface area 0.75 square meter per gram). The catalyst particle size is 60 to 100 mesh (in. 5. Sieve Series). The performance of the catalyst using the equipment described above is presented in Table X. Reactor D is used with 5.0 grams of catalyst.

## TABLE X

| Temp °C | CH$_4$ C % | Sel % | Yield % | =/- | CO$_2$/CO | CH$_4$ in Mole % | O$_2$ in Mole % | Time Min. | GHSV hr-1 | EC1 ppmv |
|---|---|---|---|---|---|---|---|---|---|---|
| 750 | 47.8 | 51.7 | 24.3 | 15.1 | 1.5 | 10.2 | 5.2 | 80 | 878 | 10 |
| 750 | 46.5 | 52.6 | 24.3 | 9.8 | 2.5 | 10.2 | 5.2 | 170 | 1171 | 10 |
| 750 | 45.7 | 51.9 | 23.8 | 8.8 | 2.9 | 10.2 | 5.2 | 260 | 1171 | 10 |
| 750 | 36.8 | 45.7 | 16.6 | 3.5 | 1.5 | 10.5 | 5.2 | 2825 | 1171 | 10 |
| 750 | 36.0 | 45.7 | 16.4 | 3.4 | 1.4 | 10.4 | 5.0 | 2915 | 1171 | 30 |
| 750 | 35.1 | 46.5 | 16.2 | 3.3 | 1.2 | 10.4 | 5.0 | 3005 | 1171 | 30 |
| 750 | 29.5 | 45.8 | 13.6 | 3.2 | 0.6 | 10.5 | 5.1 | 6740 | 1171 | 30 |
| 750 | 32.8 | 42.0 | 13.6 | 3.2 | 0.8 | 10.3 | 5.3 | 6875 | 1171 | 0 |
| 750 | 33.8 | 41.9 | 13.9 | 3.1 | 0.9 | 10.3 | 5.3 | 6920 | 1171 | 0 |
| 750 | 34.8 | 40.5 | 13.8 | 3.2 | 1.0 | 10.3 | 5.3 | 7100 | 1171 | 0 |
| 750 | 32.1 | 42.0 | 13.4 | 3.0 | 0.9 | 10.7 | 5.3 | 7190 | 1171 | 200 |
| 750 | 28.1 | 48.2 | 13.6 | 3.9 | 0.2 | 10.7 | 5.3 | 7325 | 1171 | 200 |
| 750 | 31.0 | 49.6 | 15.3 | 5.1 | 0.2 | 10.9 | 5.3 | 7820 | 1171 | 200 |
| 750 | 30.6 | 49.4 | 15.0 | 5.1 | 0.2 | 10.7 | 5.4 | 8315 | 1171 | 200 |
| 750 | 30.7 | 46.4 | 14.2 | 4.3 | 0.3 | 10.4 | 5.4 | 8405 | 1171 | 100 |
| 750 | 30.3 | 46.6 | 14.1 | 4.3 | 0.3 | 10.4 | 5.4 | 8495 | 1171 | 100 |
| 750 | 30.3 | 46.6 | 14.1 | 4.2 | 0.3 | 10.4 | 5.4 | 8585 | 1171 | 100 |

## TABLE X (continued)

| Temp °C | CH₄ C % | Sel % | Yield % | =/- | CO₂/CO | CH₄ in Mole % | O₂ in Mole % | Time Min. | GHSV hr-1 | EC1 ppmv |
|---|---|---|---|---|---|---|---|---|---|---|
| 750 | 31.8 | 50.3 | 15.9 | 6.3 | 0.1 | 10.9 | 5.2 | 8675 | 1171 | 400 |
| 750 | 32.3 | 49.9 | 16.1 | 6.5 | 0.1 | 10.9 | 5.2 | 8765 | 1171 | 400 |
| 750 | 33.9 | 49.9 | 16.7 | 6.9 | 0.1 | 10.9 | 5.2 | 8990 | 1171 | 400 |
| 750 | 32.9 | 48.9 | 16.1 | 6.9 | 0.1 | 11.2 | 5.2 | 9620 | 1171 | 400 |

## EXAMPLE 10

A 4.77 weight percent barium carbonate (3.49 weight percent barium) on alpha aluminum oxide catalyst is prepared by the general procedure B set forth above using 0.7519 gram of barium carbonate (from Johnson Matthey/AESAR Group, Seabrook, NH, Lot number S96252B, 99.997 weight percent) and 15 grams of alpha aluminum oxide (from Norton Company, Akron, OH, Sample number 8883118, Type SA 5451, B. E. T. surface area 0.27 square meter per gram). The catalyst is calcined at 850 ° C for 4 hours. The catalyst particle size is 60 to 100 mesh (U. S. Sieve Series). The performance of the catalyst using the equipment and procedures described above is presented in Table XI. Reactor D is used with 5 grams of catalyst.

TABLE XI

| Temp °C | CH₄ C % | Sel % | Yield % | =/- | CH₄ in Mole % | O₂ in Mole % | Time Min. | GHSV hr-l | ECI ppmv |
|---|---|---|---|---|---|---|---|---|---|
| 750 | 44.2 | 45.1 | 19.5 | 3.0 | 10.1 | 5.2 | 21980 | 1304 | 7 |
| 750 | 43.8 | 43.8 | 18.8 | 3.0 | 10.3 | 5.3 | 24185 | 1304 | 7 |
| 750 | 36.0 | 50.3 | 17.8 | 2.1 | 10.3 | 5.3 | 24275 | 1957 | 7 |
| 750 | 35.7 | 50.8 | 17.7 | 2.0 | 10.3 | 5.3 | 24365 | 1957 | 7 |
| 775 | 47.1 | 44.5 | 20.4 | 3.5 | 10.3 | 5.3 | 24455 | 1957 | 7 |
| 775 | 47.5 | 45.0 | 20.7 | 3.5 | 10.3 | 5.3 | 24530 | 1957 | 7 |
| 775 | 46.0 | 46.0 | 20.5 | 3.2 | 10.3 | 5.3 | 24635 | 2191 | 7 |
| 775 | 45.5 | 45.7 | 20.4 | 3.2 | 10.3 | 5.2 | 24770 | 2191 | 7 |

EXAMPLE 11 (comparative)

A 5.02 weight percent barium chloride (3.49 weight percent barium) on alpha aluminum oxide catalyst is prepared by the general procedure A set forth above using 0.317 grams of barium chloride (from Johnson Matthey/AESAR Group, Seabrook, NH, Lot number S94730, 99.9999 weight percent) and 6 grams of alpha aluminum oxide (from Norton Company, Akron, OH, Sample number 8855001). The catalyst is calcined at 850°C for 2 hours. The catalyst particle size is 18 to 60 mesh (U. S. Sieve Series). The performance of the catalyst using the equipment and procedures described above is presented in Table XII. Reactor D is used with 5 grams of catalyst.

TABLE XII

| Temp °C | CH₄ C % | Sel % | Yield % | =/- | CH₄ in Mole % | O₂ in Mole % | Time Min. | GHSV hr-l | ECI ppmv |
|---|---|---|---|---|---|---|---|---|---|
| 750 | 41.5 | 62.1 | 25.3 | 5.5 | 10.6 | 5.1 | 35 | 2341 | 0 |
| 750 | 31.4 | 69.1 | 21.3 | 3.2 | 10.6 | 5.1 | 90 | 2341 | 0 |
| 750 | 20.0 | 73.0 | 14.5 | 1.7 | 10.4 | 5.1 | 215 | 2341 | 10 |
| 750 | 13.3 | 73.0 | 9.6 | 1.0 | 10.4 | 5.1 | 350 | 2341 | 10 |
| 750 | 8.0 | 66.5 | 5.3 | 0.6 | 10.4 | 5.2 | 5255 | 2341 | 10 |
| 750 | 6.9 | 66.1 | 4.6 | 0.6 | 10.3 | 5.2 | 5390 | 2341 | 200 |
| 750 | 7.0 | 66.0 | 4.6 | 0.6 | 10.3 | 5.2 | 5480 | 2341 | 200 |

EXAMPLE 12 (Comparative)

A 8 weight percent hydrated barium bromide (7 weight percent barium bromide, 3.5 weight percent barium) on aluminum oxide catalyst is prepared by the general procedure A set forth above using 0.5 gram of hydrated barium bromide (2 waters of hydration, from Johnson Matthey/AESAR Group, Seabrook, NH, Lot number S96383, 99.999 weight percent) and 6 grams of aluminum oxide (from Norton Company, Akron, OH, Sample number 8883118, Type SA 5451, B. E. T. surface area 0.27 square meter per gram). The catalyst is calcined at 850°C for 2 hours. The catalyst particle size is 60 to 100 mesh (U. S. Sieve Series). The catalyst is run for 2575 minutes in the absence of added halogen compound. The catalyst is then evaluated for oxidative coupling of methane, using the equipment and procedures described above, and the results are set forth in Table XIII. Ethyl bromide (EBr) is used as the halogen component. Reactor D is used with 5.0 grams of catalyst.

TABLE XIII

| Temp °C | CH₄ C % | Sel % | Yield % | =/- | CO₂/CO | CH₄ in Mole % | O₂ in Mole % | Time Min. | GHSV hr-l | EBr ppmv |
|---|---|---|---|---|---|---|---|---|---|---|
| 750 | 34.3 | 42.4 | 14.8 | 3.4 | 1.3 | 10.2 | 5.2 | 35 | 2182 | 5 |
| 750 | 33.0 | 47.4 | 15.6 | 3.9 | 1.2 | 10.2 | 5.2 | 110 | 2182 | 5 |
| 750 | 35.7 | 41.0 | 14.5 | 2.9 | 1.5 | 10.5 | 5.1 | 4285 | 2182 | 5 |
| 750 | 36.2 | 40.5 | 14.7 | 3.1 | 1.4 | 9.9 | 5.2 | 4375 | 2182 | 10 |
| 750 | 36.7 | 40.8 | 14.9 | 3.3 | 1.4 | 9.9 | 5.2 | 4510 | 2182 | 10 |
| 750 | 37.3 | 40.5 | 15.1 | 3.5 | 1.4 | 9.8 | 5.1 | 5320 | 2182 | 10 |
| 750 | 37.4 | 40.3 | 15.1 | 3.6 | 1.4 | 9.9 | 5.2 | 5625 | 2182 | 10 |
| 750 | 38.4 | 40.7 | 15.5 | 4.0 | 1.3 | 10.0 | 5.1 | 5725 | 2182 | 17 |
| 750 | 38.5 | 40.3 | 15.5 | 4.1 | 1.3 | 10.0 | 5.1 | 5815 | 2182 | 17 |
| 750 | 38.6 | 40.6 | 15.6 | 4.2 | 1.3 | 10.0 | 5.1 | 5995 | 2182 | 17 |
| 750 | 37.0 | 40.0 | 14.9 | 3.8 | 1.4 | 10.2 | 5.1 | 6105 | 2182 | 1 |
| 750 | 35.5 | 39.8 | 14.1 | 3.0 | 1.5 | 10.2 | 5.1 | 6220 | 2182 | 1 |

EXAMPLE 13 (Comparative)

A 6 weight percent barium nitrate (3.5 weight percent barium) on alpha aluminum oxide catalyst is prepared by the general procedure B set forth above using 0.40 gram of barium nitrate (from Johnson Matthey/AESAR Group, Seabrook, NH, Lot number S96252B, 99.997 weight percent) and 6 grams of alpha aluminum oxide (from Norton Company, Akron, OH, Sample number 8883118, Type SA 5451, B. E. T. surface area 0.27 square meter per gram). The catalyst is calcined at 850°C for 4 hours. The catalyst particle size is 60 to 100 mesh (in. 5. Sieve Series). The performance of the catalyst using the equipment described above is presented in Table XIV. Reactor D is used with 5.0 grams of catalyst.

TABLE XIV

| Temp °C | CH₄ C % | Sel % | Yield % | =/- | CO₂/CO | CH₄ in Mole % | O₂ in Mole % | Time Min. | GHSV hr-l | ECl ppmv |
|---|---|---|---|---|---|---|---|---|---|---|
| 750 | 17.5 | 13.8 | 2.4 | 0.6 | 2.3 | 10.4 | 5.2 | 105 | 2133 | 0 |
| 750 | 17.1 | 13.2 | 2.3 | 0.6 | 2.1 | 10.4 | 5.2 | 330 | 2133 | 0 |
| 750 | 16.2 | 9.1 | 1.5 | 0.6 | 1.9 | 10.4 | 5.3 | 3795 | 2133 | 0 |
| 750 | 26.6 | 52.0 | 14.2 | 3.0 | 0.4 | 10.4 | 5.1 | 3930 | 2133 | 10 |
| 750 | 19.0 | 50.5 | 9.8 | 1.7 | 0.5 | 10.4 | 5.1 | 4020 | 2133 | 10 |
| 750 | 17.7 | 47.8 | 8.5 | 1.5 | 0.5 | 10.4 | 5.1 | 4110 | 2133 | 10 |
| 750 | 14.6 | 38.6 | 5.7 | 1.0 | 0.6 | 10.3 | 5.1 | 4300 | 2133 | 2 |
| 750 | 13.9 | 35.6 | 5.0 | 1.0 | 0.6 | 10.3 | 5.1 | 4380 | 2133 | 2 |
| 750 | 9.4 | 19.6 | 1.9 | 0.5 | 0.6 | 10.3 | 5.1 | 5820 | 2133 | 2 |
| 750 | 12.4 | 27.5 | 3.4 | 0.8 | 0.5 | 10.4 | 5.2 | 5910 | 2133 | 200 |
| 750 | 12.8 | 28.4 | 3.7 | 0.8 | 0.5 | 10.4 | 5.2 | 6000 | 2133 | 200 |
| 750 | 27.5 | 57.5 | 16.0 | 3.3 | 0.3 | 10.3 | 5.2 | 6405 | 2133 | 200 |
| 750 | 26.8 | 57.5 | 15.4 | 3.2 | 0.3 | 10.4 | 5.2 | 6900 | 2133 | 200 |
| 750 | 33.2 | 48.8 | 16.3 | 4.6 | 0.3 | 10.4 | 5.2 | 6935 | 2133 | 200 |
| 750 | 33.3 | 49.6 | 16.5 | 4.5 | 0.3 | 10.4 | 5.2 | 6965 | 2133 | 200 |
| 750 | 25.2 | 56.5 | 14.2 | 3.0 | 0.3 | 10.4 | 5.2 | 7010 | 3000 | 200 |
| 750 | 24.8 | 56.8 | 14.1 | 2.9 | 0.3 | 10.4 | 5.2 | 7050 | 3000 | 200 |
| 750 | 19.6 | 64.1 | 12.5 | 2.1 | 0.3 | 10.4 | 5.2 | 7125 | 3000 | 200 |
| 750 | 19.2 | 65.1 | 12.4 | 2.0 | 0.3 | 10.4 | 5.2 | 7215 | 3000 | 200 |
| 750 | 13.2 | 69.1 | 9.1 | 1.4 | 0.2 | 10.4 | 5.2 | 8340 | 3000 | 200 |
| 750 | 32.1 | 51.0 | 16.4 | 4.6 | 0.2 | 10.4 | 5.2 | 8415 | 1067 | 200 |
| 750 | 31.5 | 51.4 | 16.3 | 4.6 | 0.3 | 10.4 | 5.2 | 8445 | 1067 | 200 |

EXAMPLE 14 (comparative)

A barium oxide aluminum oxide catalyst (available from Alfa, Danvers, Massachusetts, as 3BaO•Al₂O₃ - (barium aluminate) Lot number K18G, 99.99 weight percent) is calcined at 850°C for 2 hours. By powder X-ray analysis, the catalyst appears to contain barium oxide, barium carbonate and barium aluminate phases. The performance of 2.0 grams of 60 to 100 mesh (U. S. Sieve Series) catalyst using the equipment and procedures described above, with Reactor D, is presented in Table XV.

33

TABLE XV

| Temp °C | CH₄ C % | Sel % | Yield % | =/- | CO₂/CO | CH₄ in Mole % | O₂ in Mole % | Time Min. | GHSV hr-l | ECl ppmv |
|---|---|---|---|---|---|---|---|---|---|---|
| 750 | 37.7 | 25.5 | 9.5 | 1.7 | 6.1 | 9.6 | 4.8 | 55 | 3429 | 10 |
| 750 | 39.0 | 28.2 | 10.8 | 1.8 | 6.1 | 9.6 | 4.8 | 155 | 3429 | 10 |
| 750 | 41.9 | 35.6 | 14.7 | 2.0 | 6.3 | 9.2 | 4.6 | 1145 | 3429 | 10 |
| 740 | 41.1 | 33.8 | 13.7 | 2.0 | 5.8 | 9.2 | 4.6 | 1235 | 3429 | 10 |
| 720 | 38.3 | 26.0 | 10.0 | 1.9 | 4.4 | 9.2 | 4.6 | 1325 | 3429 | 10 |
| 700 | 32.6 | 15.8 | 5.2 | 1.4 | 2.6 | 9.2 | 4.6 | 1415 | 3429 | 10 |
| 700 | 32.2 | 15.7 | 5.1 | 1.4 | 2.7 | 9.2 | 4.6 | 1505 | 3429 | 10 |
| 700 | 32.6 | 15.4 | 5.0 | 1.4 | 2.6 | 9.6 | 4.9 | 2405 | 3429 | 10 |
| 700 | 32.6 | 15.3 | 5.0 | 1.4 | 2.6 | 9.5 | 4.8 | 2620 | 3429 | 25 |
| 700 | 32.5 | 14.8 | 4.8 | 1.4 | 2.5 | 9.5 | 4.8 | 2765 | 3429 | 25 |
| 720 | 39.1 | 26.0 | 10.0 | 1.9 | 4.3 | 9.5 | 4.8 | 2840 | 3429 | 25 |
| 720 | 39.2 | 26.2 | 10.1 | 1.9 | 4.3 | 9.5 | 4.8 | 2945 | 3429 | 25 |
| 720 | 39.0 | 26.3 | 10.1 | 1.9 | 4.3 | 9.6 | 4.9 | 3035 | 3429 | 200 |
| 720 | 39.1 | 26.9 | 10.4 | 1.9 | 4.2 | 9.6 | 4.9 | 3530 | 3429 | 200 |
| 720 | 39.2 | 26.9 | 10.4 | 1.9 | 4.2 | 9.6 | 4.9 | 3710 | 3429 | 200 |
| 720 | 38.7 | 26.1 | 10.0 | 1.8 | 3.9 | 9.4 | 4.6 | 4010 | 3429 | 200 |
| ·750 | 42.4 | 36.2 | 15.1 | 2.0 | 6.2 | 9.4 | 4.6 | 4070 | 3429 | 200 |
| 750 | 42.6 | 36.6 | 15.3 | 2.0 | 6.2 | 9.4 | 4.6 | 4130 | 3429 | 200 |
| 750 | 42.9 | 36.5 | 15.3 | 2.0 | 5.9 | 9.4 | 4.6 | 4220 | 3429 | 200 |

EXAMPLE 15

A 99.997 weight percent barium carbonate on alpha aluminum oxide catalyst is prepared by the general procedure B set forth above using 8 grams of barium carbonate (from Johnson Matthey/AESAR Group, Seabrook, NH, Lot number S97090R, 99.997 weight percent) except 100 milliliters of water are used. The catalyst is calcined at 850°C for 2 hours. The catalyst particle size is 40 to 100 mesh (U. S. Sieve Series). The performance of the catalyst using the equipment and procedures described above is presented in Table XVI. Reactor D is used with 5 grams of catalyst.

## TABLE XVI

| Temp °C | CH₄ C % | Sel % | Yield % | =/- | CH₄ in Mole % | O₂ in Mole % | Time Min. | GHSV hr-1 | ECl ppmv |
|---|---|---|---|---|---|---|---|---|---|
| 750 | 36.2 | 31.6 | 11.3 | 1.7 | 10.2 | 5.1 | 30 | 2233 | 8 |
| 750 | 30.5 | 27.6 | 8.3 | 1.3 | 10.2 | 5.1 | 85 | 2233 | 8 |
| 750 | 28.9 | 26.7 | 7.6 | 1.3 | 10.2 | 5.1 | 165 | 2233 | 8 |
| 750 | 28.0 | 25.8 | 7.2 | 1.2 | 10.2 | 5.1 | 255 | 2233 | 8 |
| 750 | 29.5 | 32.4 | 9.5 | 1.6 | 10.2 | 5.1 | 430 | 2233 | 8 |
| 750 | 34.9 | 40.6 | 14.0 | 2.0 | 10.2 | 5.2 | 655 | 2233 | 8 |
| 750 | 36.0 | 43.3 | 15.4 | 2.1 | 10.2 | 5.2 | 835 | 2233 | 8 |
| 750 | 36.3 | 43.8 | 15.7 | 2.1 | 10.2 | 5.2 | 1015 | 2233 | 8 |
| 750 | 36.2 | 44.0 | 15.8 | 2.1 | 10.2 | 5.1 | 1150 | 2233 | 8 |
| 750 | 36.1 | 43.6 | 15.7 | 2.1 | 10.2 | 5.1 | 1240 | 2233 | 8 |
| 750 | 35.8 | 44.3 | 15.7 | 2.1 | 10.2 | 5.1 | 1330 | 2233 | 8 |
| 750 | 35.7 | 44.5 | 15.8 | 2.0 | 10.2 | 5.1 | 1420 | 2233 | 8 |
| 750 | 35.6 | 44.6 | 15.7 | 2.0 | 10.2 | 5.1 | 1510 | 2233 | 8 |
| 750 | 33.3 | 45.8 | 15.2 | 1.9 | 10.1 | 5.2 | 2275 | 2233 | 8 |
| 750 | 32.9 | 46.0 | 15.1 | 1.9 | 10.1 | 5.2 | 2455 | 2233 | 8 |
| 750 | 30.9 | 47.1 | 14.5 | 1.7 | 10.1 | 5.1 | 3535 | 2233 | 8 |
| 750 | 30.3 | 47.6 | 14.4 | 1.7 | 10.1 | 5.1 | 3715 | 2233 | 8 |
| 750 | 29.3 | 48.2 | 14.1 | 1.6 | 10.1 | 5.1 | 4570 | 2233 | 8 |

EP 0 418 972 A1

TABLE XVI (continued)

| Temp °C | CH4 C % | Sel % | Yield % | =/- | CH4 in Mole % | O2 in Mole % | Time Min. | GHSV hr-1 | EC1 ppmv |
|---|---|---|---|---|---|---|---|---|---|
| 750 | 28.2 | 48.8 | 13.7 | 1.6 | 10.2 | 5.1 | 5470 | 2233 | 8 |
| 750 | 28.5 | 48.9 | 13.8 | 1.6 | 10.2 | 5.1 | 5650 | 2233 | 8 |
| 750 | 28.2 | 48.9 | 13.7 | 1.5 | 10.2 | 5.1 | 5830 | 2233 | 8 |
| 750 | 27.5 | 49.1 | 13.4 | 1.5 | 10.1 | 5.1 | 6190 | 2233 | 8 |
| 750 | 27.5 | 49.4 | 13.5 | 1.5 | 10.2 | 5.1 | 6415 | 2233 | 8 |
| 750 | 26.3 | 49.6 | 13.1 | 1.5 | 10.1 | 5.1 | 7270 | 2233 | 8 |
| 750 | 26.7 | 49.5 | 13.1 | 1.5 | 10.1 | 5.1 | 7360 | 2233 | 8 |
| 750 | 26.2 | 50.2 | 13.1 | 1.4 | 10.1 | 5.2 | 8350 | 2233 | 8 |
| 750 | 25.8 | 50.0 | 12.9 | 1.4 | 10.0 | 5.1 | 8890 | 2233 | 8 |
| 750 | 25.9 | 50.7 | 12.9 | 1.4 | 10.3 | 5.0 | 11320 | 2233 | 8 |
| 750 | 25.9 | 50.7 | 12.9 | 1.4 | 10.3 | 5.0 | 11410 | 2233 | 8 |

EXAMPLE 16

A 5 weight percent barium carbonate (3.5 weight percent barium) on titanium dioxide catalyst is prepared by the general procedure B set forth above using 0.3 gram of barium carbonate (from Johnson Matthey/AESAR Group, Seabrook, NH, Lot number S96252B, 99.997 weight percent) and 6.0 grams of titanium dioxide (from Johnson Matthey/AESAR Group, Seabrook, NH, Lot number S96579, 99.995 weight percent) except 40 milliliters of water are used. The catalyst is calcined at 850° C for 4 hours. The catalyst particle size is 60 to 100 mesh (U. S. Sieve Series). The performance of the catalyst using the equipment and procedures described above is presented in Table XVII. Reactor D is used with 5.0 grams of catalyst. As can be seen, the amount of halogen component has a demonstrable effect on the performance of the catalyst.

EP 0 418 972 A1

## TABLE XVII

| Temp °C | CH$_4$C % | Sel % | Yield % | =/- | CO$_2$/CO | CH$_4$ in Mole % | O$_2$ in Mole % | Time Min. | GHSV hr-1 | EC1 ppmv |
|---|---|---|---|---|---|---|---|---|---|---|
| 750 | 22.0 | 8.5 | 1.9 | 0.7 | 0.9 | 10.4 | 5.1 | 30 | 2743 | 6 |
| 750 | 21.0 | 9.2 | 1.9 | 0.8 | 0.8 | 10.4 | 5.1 | 120 | 2743 | 6 |
| 750 | 17.4 | 13.2 | 2.3 | 0.7 | 0.7 | 10.2 | 4.9 | 4845 | 2743 | 6 |
| 750 | 15.9 | 14.1 | 2.2 | 0.7 | 0.7 | 10.0 | 5.2 | 7050 | 2743 | 6 |
| 750 | 30.3 | 40.6 | 12.4 | 4.9 | 0.3 | 10.2 | 5.2 | 8850 | 2743 | 500 |
| 750 | 31.1 | 49.2 | 15.3 | 5.4 | 0.3 | 10.2 | 5.2 | 8985 | 2743 | 500 |
| 750 | 30.9 | 59.4 | 18.1 | 5.6 | 0.3 | 10.3 | 5.0 | 9570 | 2743 | 500 |
| 750 | 31.4 | 59.2 | 18.4 | 5.9 | 0.3 | 10.2 | 5.2 | 9630 | 2743 | 500 |
| 750 | 30.9 | 60.1 | 18.4 | 5.8 | 0.3 | 10.2 | 5.2 | 9720 | 2743 | 500 |
| 750 | 31.1 | 63.2 | 19.1 | 5.6 | 0.3 | 10.2 | 4.9 | 11430 | 2743 | 500 |
| 750 | 38.8 | 54.5 | 20.5 | 8.4 | 0.3 | 10.2 | 4.9 | 11520 | 1714 | 500 |
| 750 | 38.9 | 53.9 | 20.4 | 8.5 | 0.3 | 10.2 | 4.9 | 11610 | 1714 | 500 |
| 750 | 37.9 | 53.7 | 20.1 | 8.4 | 0.3 | 10.2 | 5.1 | 12915 | 1714 | 500 |
| 750 | 34.3 | 54.6 | 18.5 | 5.9 | 0.5 | 10.2 | 4.9 | 13005 | 1714 | 250 |
| 750 | 34.3 | 53.9 | 18.2 | 5.8 | 0.5 | 10.2 | 4.9 | 13095 | 1714 | 250 |
| 750 | 33.4 | 53.3 | 17.7 | 5.7 | 0.5 | 10.3 | 5.3 | 13815 | 1714 | 250 |
| 750 | 33.9 | 52.6 | 17.7 | 5.8 | 0.5 | 10.3 | 5.2 | 13950 | 1714 | 250 |
| 750 | 34.3 | 52.3 | 17.7 | 5.8 | 0.5 | 10.3 | 5.2 | 14040 | 1714 | 250 |

EP 0 418 972 A1

## TABLE XVII (continued)

| Temp °C | CH$_4$ C % | Sel % | Yield % | =/- | CO$_2$/CO | CH$_4$ in Mole % | O$_2$ in Mole % | Time Min. | GHSV hr-1 | EC1 ppmv |
|---|---|---|---|---|---|---|---|---|---|---|
| 750 | 34.9 | 52.0 | 17.8 | 5.8 | 0.5 | 10.3 | 5.2 | 14220 | 1714 | 250 |
| 750 | 34.9 | 52.0 | 17.8 | 5.8 | 0.5 | 10.3 | 5.2 | 14310 | 1714 | 250 |
| 750 | 30.9 | 61.6 | 18.7 | 5.7 | 0.3 | 10.2 | 5.1 | 14385 | 2743 | 500 |
| 750 | 30.5 | 61.9 | 18.6 | 5.6 | 0.3 | 10.2 | 5.1 | 14460 | 2743 | 500 |
| 750 | 23.9 | 65.5 | 15.5 | 4.0 | 0.3 | 16.1 | 5.4 | 14535 | 2743 | 500 |
| 750 | 23.9 | 65.5 | 15.5 | 4.0 | 0.3 | 16.1 | 5.4 | 14625 | 2743 | 500 |
| 750 | 24.8 | 66.0 | 16.0 | 3.9 | 0.3 | 16.0 | 5.1 | 16020 | 2743 | 500 |
| 750 | 23.3 | 65.9 | 15.3 | 3.9 | 0.3 | 16.3 | 5.2 | 18225 | 2743 | 500 |
| 750 | 30.3 | 58.2 | 17.3 | 5.9 | 0.3 | 16.2 | 5.1 | 18360 | 1714 | 500 |
| 750 | 30.4 | 58.7 | 17.4 | 5.9 | 0.4 | 16.2 | 5.1 | 18450 | 1714 | 500 |
| 750 | 44.9 | 48.8 | 21.8 | 11.3 | 0.3 | 7.3 | 5.1 | 18540 | 1714 | 500 |
| 750 | 44.1 | 49.5 | 21.9 | 11.3 | 0.3 | 7.3 | 5.1 | 18610 | 1714 | 500 |
| 750 | 34.2 | 59.1 | 20.5 | 7.3 | 0.3 | 7.3 | 5.1 | 18690 | 2743 | 500 |
| 750 | 34.6 | 58.2 | 20.5 | 7.4 | 0.3 | 7.3 | 5.1 | 18780 | 2743 | 500 |
| 750 | 37.7 | 58.1 | 21.7 | 8.0 | 0.3 | 6.2 | 5.1 | 18855 | 2743 | 500 |
| 750 | 38.0 | 58.2 | 21.9 | 8.0 | 0.3 | 6.2 | 5.1 | 18945 | 2743 | 500 |
| 750 | 37.2 | 60.8 | 22.2 | 7.5 | 0.3 | 6.4 | 5.1 | 19485 | 2743 | 500 |
| 750 | 36.9 | 60.9 | 22.1 | 7.4 | 0.3 | 6.4 | 5.1 | 19665 | 2743 | 500 |
| 750 | 37.4 | 59.0 | 21.9 | 7.9 | 0.3 | 6.4 | 4.9 | 19800 | 2743 | 500 |
| 750 | 38.2 | 58.5 | 22.0 | 8.1 | 0.3 | 6.4 | 4.9 | 19890 | 2743 | 500 |

**TABLE XVII (continued)**

| Temp °C | CH₄ C % | Sel % | Yield % | =/- | CO₂/CO | CH₄ in Mole % | O₂ in Mole % | Time Min. | GHSV hr⁻¹ | ECl ppmv |
|---|---|---|---|---|---|---|---|---|---|---|
| 750 | 38.9 | 58.5 | 22.2 | 8.2 | 0.3 | 6.4 | 4.9 | 19980 | 2743 | 500 |
| 750 | 38.1 | 58.9 | 22.1 | 8.1 | 0.3 | 6.4 | 5.1 | 20070 | 2743 | 500 |
| 770 | 48.0 | 47.8 | 23.1 | 11.1 | 0.3 | 6.4 | 5.1 | 20160 | 2743 | 500 |
| 730 | 30.3 | 63.3 | 18.8 | 5.8 | 0.2 | 6.4 | 5.1 | 20210 | 2743 | 500 |
| 750 | 39.0 | 58.2 | 22.2 | 8.2 | 0.3 | 6.4 | 5.1 | 20295 | 2743 | 500 |
| 750 | 38.6 | 58.7 | 22.2 | 8.1 | 0.3 | 6.4 | 5.1 | 20385 | 2743 | 500 |
| 750 | 38.8 | 58.4 | 22.3 | 8.2 | 0.3 | 6.3 | 4.8 | 20565 | 2743 | 500 |
| 750 | 37.8 | 58.5 | 22.0 | 8.1 | 0.3 | 6.4 | 5.1 | 21105 | 2743 | 500 |
| 750 | 47.1 | 47.4 | 22.6 | 12.8 | 0.3 | 6.2 | 5.0 | 21240 | 1714 | 500 |
| 750 | 47.5 | 47.2 | 22.5 | 12.8 | 0.3 | 6.2 | 5.0 | 21330 | 1714 | 500 |

EXAMPLE 17 (Comparative)

A 5 weight percent barium chloride (3.5 weight percent barium) on titanium dioxide catalyst is prepared by the general procedure A set forth above using 0.27 gram of barium chloride (from Johnson Matthey/AESAR Group, Seabrook, NH, Lot number S94730, 99.9999 weight percent) and 5 grams of titanium dioxide (from Johnson Matthey/AESAR Group, Seabrook, NH, Lot number S96579, 99.995 weight percent, B. E. T. surface area 0.82 square meter per gram). The catalyst particle size is 30 to 60 mesh (U. S. Sieve Series). The performance of the catalyst using the equipment and procedures described above is presented in Table XVIII. Reactor D is used with 5 grams of catalyst.

## TABLE XVIII

| Temp $^\circ$C | CH₄ C % | Sel % | Yield % | =/- | CO₂/CO | CH₄ in Mole % | O₂ in Mole % | Time Min. | GHSV hr-l | ECI ppmv |
|---|---|---|---|---|---|---|---|---|---|---|
| 750 | 38.2 | 47.9 | 18.1 | 11.0 | 0.4 | 9.8 | 5.2 | 30 | 878 | 0 |
| 750 | 38.1 | 46.2 | 17.4 | 10.6 | 0.4 | 9.8 | 5.2 | 140 | 878 | 0 |
| 750 | 37.0 | 44.9 | 16.5 | 9.7 | 0.5 | 9.8 | 5.2 | 230 | 878 | 0 |
| 750 | 36.4 | 42.8 | 15.5 | 8.8 | 0.6 | 9.8 | 5.2 | 320 | 878 | 0 |
| 750 | 35.5 | 41.0 | 14.5 | 8.0 | 0.7 | 9.8 | 5.2 | 410 | 878 | 0 |
| 750 | 36.0 | 38.4 | 13.6 | 6.5 | 0.8 | 9.8 | 4.7 | 590 | 878 | 0 |
| 750 | 36.7 | 35.9 | 12.7 | 5.5 | 0.9 | 9.8 | 4.7 | 770 | 878 | 0 |
| 750 | 34.8 | 32.8 | 11.3 | 4.9 | 1.0 | 9.8 | 4.7 | 950 | 878 | 0 |
| 750 | 33.2 | 31.3 | 10.4 | 4.5 | 1.1 | 9.7 | 4.6 | 1085 | 878 | 0 |
| 750 | 33.7 | 30.2 | 10.1 | 4.4 | 1.1 | 9.7 | 4.6 | 1175 | 878 | 0 |
| 750 | 33.1 | 29.5 | 9.8 | 4.2 | 1.1 | 9.7 | 4.6 | 1265 | 878 | 0 |
| 750 | 33.2 | 28.7 | 9.5 | 4.0 | 1.2 | 9.7 | 4.6 | 1355 | 878 | 0 |
| 750 | 33.0 | 28.2 | 9.3 | 3.9 | 1.2 | 9.7 | 4.6 | 1400 | 878 | 0 |
| 750 | 39.1 | 41.7 | 16.3 | 10.3 | 0.5 | 10.1 | 4.6 | 1535 | 878 | 500 |
| 750 | 39.0 | 42.2 | 16.5 | 10.5 | 0.5 | 10.1 | 4.6 | 1670 | 878 | 500 |
| 750 | 39.0 | 42.4 | 16.6 | 10.5 | 0.5 | 10.1 | 4.6 | 1850 | 878 | 500 |
| 750 | 39.7 | 42.5 | 16.8 | 10.5 | 0.5 | 10.4 | 4.9 | 2030 | 878 | 500 |
| 750 | 39.2 | 42.3 | 16.6 | 10.5 | 0.5 | 10.4 | 4.9 | 2210 | 878 | 500 |
| 750 | 38.9 | 42.5 | 16.6 | 10.5 | 0.5 | 10.4 | 4.9 | 2390 | 878 | 500 |
| 750 | 38.4 | 42.7 | 16.5 | 10.4 | 0.5 | 10.1 | 5.1 | 2525 | 878 | 500 |

## EXAMPLE 18

A 5 weight percent barium carbonate (3.5 weight percent barium) on zirconium dioxide catalyst is prepared by the general procedure B set forth above using 0.301 gram of barium carbonate (from Johnson Matthey/AESAR Group, Seabrook, NH, Lot number 5962529, 99.997 weight percent) and 6.0 grams of zirconium dioxide (from Johnson Matthey/AESAR Group, Seabrook, NH, Lot number S96307, 99.9975 weight percent, B. E. T. surface area 0.95 square meter per gram). The catalyst is calcined at 850$^\circ$C for 2 hours. The catalyst particle size is 60 to 100 mesh (U. S. Sieve Series). Reactor D is used with 5.0 grams of catalyst diluted with 2 cubic centimeters of quartz chips of particle size of 14 to 30 mesh (U. S. Sieve Series). The results of evaluating the catalyst using the equipment and procedures described above are set forth in Table XIX. A high space velocity (5053 hr-1) is used.

EP 0 418 972 A1

## TABLE XIX

| Temp °C | CH$_4$ C % | Sel % | Yield % | =/- | CO$_2$/CO | CH$_4$ in Mole % | O$_2$ in Mole % | Time Min. | GHSV hr-1 | EC1 ppmv |
|---|---|---|---|---|---|---|---|---|---|---|
| 750 | 37.1 | 24.7 | 9.1 | 1.3 | 5.4 | 9.3 | 4.6 | 45 | 5053 | 10 |
| 750 | 36.9 | 26.1 | 9.6 | 1.3 | 4.8 | 9.3 | 4.6 | 185 | 5053 | 10 |
| 750 | 37.3 | 27.4 | 10.1 | 1.4 | 4.5 | 9.5 | 4.8 | 365 | 5053 | 10 |
| 750 | 37.2 | 28.8 | 10.6 | 1.4 | 4.4 | 9.6 | 4.8 | 590 | 5053 | 10 |
| 750 | 37.3 | 29.7 | 10.9 | 1.4 | 4.4 | 9.6 | 4.8 | 770 | 5053 | 10 |
| 750 | 37.2 | 30.2 | 11.1 | 1.4 | 4.3 | 9.6 | 4.8 | 950 | 5053 | 10 |
| 750 | 37.0 | 30.2 | 11.1 | 1.4 | 4.2 | 9.6 | 4.8 | 1085 | 5053 | 10 |
| 750 | 37.1 | 30.6 | 11.2 | 1.4 | 4.1 | 9.6 | 4.8 | 1445 | 5053 | 10 |
| 750 | 36.2 | 30.6 | 11.0 | 1.4 | 3.9 | 9.7 | 4.8 | 1805 | 5053 | 10 |
| 750 | 36.0 | 30.0 | 10.7 | 1.4 | 3.7 | 9.7 | 4.7 | 2210 | 5053 | 10 |
| 750 | 35.6 | 29.0 | 10.3 | 1.4 | 3.5 | 9.5 | 4.6 | 2525 | 5053 | 10 |
| 750 | 34.5 | 28.6 | 9.9 | 1.4 | 3.2 | 9.5 | 4.6 | 2885 | 5053 | 10 |
| 750 | 34.9 | 27.8 | 9.6 | 1.4 | 3.0 | 9.7 | 4.7 | 3245 | 5053 | 10 |
| 750 | 34.5 | 26.6 | 9.2 | 1.4 | 2.8 | 9.7 | 5.0 | 3650 | 5053 | 10 |
| 750 | 34.7 | 26.0 | 8.9 | 1.4 | 2.7 | 9.7 | 4.7 | 3965 | 5053 | 10 |
| 750 | 34.7 | 25.2 | 8.6 | 1.4 | 2.6 | 9.7 | 4.7 | 4325 | 5053 | 10 |
| 750 | 34.2 | 24.6 | 8.3 | 1.4 | 2.5 | 9.8 | 4.9 | 4685 | 5053 | 10 |
| 750 | 34.0 | 23.9 | 8.1 | 1.4 | 2.4 | 9.8 | 5.0 | 5090 | 5053 | 10 |

**TABLE XIX (continued)**

| Temp °C | CH₄ C % | Sel % | Yield % | =/- | CO₂/CO | CH₄ in Mole % | O₂ in Mole % | Time Min. | GHSV hr-1 | ECl ppmv |
|---|---|---|---|---|---|---|---|---|---|---|
| 750 | 30.3 | 34.7 | 10.5 | 2.2 | 1.1 | 9.6 | 5.1 | 6530 | 5053 | 200 |
| 750 | 29.8 | 36.0 | 10.8 | 2.5 | 0.6 | 9.5 | 5.0 | 6845 | 5053 | 200 |
| 750 | 28.1 | 42.8 | 12.0 | 2.7 | 0.4 | 9.5 | 5.0 | 7205 | 5053 | 200 |
| 750 | 27.7 | 42.9 | 11.9 | 2.8 | 0.3 | 9.5 | 4.8 | 7565 | 5053 | 200 |
| 750 | 27.8 | 42.9 | 11.9 | 2.8 | 0.3 | 9.4 | 4.8 | 7970 | 5053 | 200 |

EXAMPLE 19

A 4.77 weight percent barium carbonate (3.49 weight percent barium) on titanium dioxide catalyst is prepared by the general procedure B set forth above using 0.902 gram of barium carbonate (from Johnson Matthey/AESAR Group, Seabrook, NH, Lot number S97090R, 99.997 weight percent) and 18 grams of titanium dioxide (from Johnson Matthey/AESAR Group, Seabrook, NH, Lot number S96579, 99.995 weight percent, B. E. T. surface area 0.82 square meter per gram) except 40 milliliters of water are used. The catalyst is calcined at 850°C for 4 hours. The catalyst particle size is 30 to 60 mesh (U. S. Sieve Series). The performance of the catalyst using the equipment and procedures described above is presented in Table XX. Reactor D is used with 5 grams of catalyst. When ethyl bromide is used instead of ethyl chloride, EBr appears to the right of the ECl column.

42

TABLE XX

| Temp °C | CH$_4$ C % | Sel % | Yield % | =/- | CH$_4$ in Mole % | O$_2$ in Mole % | Time Min. | GHSV hr-1 | EC1 ppmv | EBr |
|---|---|---|---|---|---|---|---|---|---|---|
| 750 | 43.1 | 23.4 | 10.9 | 26.9 | 10.0 | 5.0 | 50 | 1935 | 250 | EBr |
| 750 | 38.4 | 28.0 | 11.4 | 14.8 | 10.0 | 5.0 | 160 | 3097 | 250 | EBr |
| 750 | 39.2 | 32.7 | 13.3 | 13.9 | 10.1 | 4.9 | 3155 | 3097 | 250 | EBr |
| 750 | 38.2 | 32.8 | 13.2 | 13.7 | 10.0 | 5.0 | 3920 | 3097 | 250 | EBr |
| 750 | 30.3 | 37.1 | 11.5 | 5.8 | 9.9 | 4.9 | 4100 | 3097 | 50 | EBr |
| 750 | 30.4 | 37.1 | 11.5 | 5.9 | 9.9 | 4.9 | 4190 | 3097 | 50 | EBr |
| 750 | 30.5 | 37.3 | 11.5 | 5.7 | 9.9 | 4.9 | 4245 | 3097 | 50 | EBr |
| 750 | 23.1 | 52.1 | 12.2 | 3.1 | 9.7 | 4.9 | 4340 | 3097 | 250 | |
| 750 | 24.0 | 51.5 | 12.5 | 3.4 | 9.7 | 4.9 | 4415 | 3097 | 250 | |
| 750 | 22.9 | 59.0 | 13.5 | 2.9 | 9.6 | 4.9 | 5360 | 3097 | 250 | |
| 750 | 21.8 | 59.3 | 13.0 | 2.8 | 9.7 | 4.9 | 5685 | 3097 | 250 | |
| 750 | 17.2 | 50.4 | 8.6 | 1.4 | 9.6 | 4.9 | 5770 | 3097 | 50 | |
| 750 | 16.8 | 49.4 | 8.2 | 1.4 | 9.6 | 4.9 | 5835 | 3097 | 50 | |
| 750 | 15.9 | 46.4 | 7.4 | 1.4 | 9.6 | 5.0 | 6810 | 3097 | 50 | |
| 750 | 14.8 | 19.4 | 2.8 | 0.5 | 9.9 | 5.0 | 6945 | 3097 | 0 | |
| 750 | 15.9 | 13.4 | 2.1 | 0.5 | 9.9 | 5.0 | 7080 | 3097 | 0 | |
| 750 | 16.7 | 12.1 | 2.0 | 0.5 | 9.9 | 5.0 | 7155 | 3097 | 0 | |
| 750 | 25.4 | 55.4 | 14.3 | 4.0 | 9.8 | 5.0 | 7250 | 3097 | 0 | |

**TABLE XX (continued)**

| Temp °C | CH₄ C % | Sel % | Yield % | =/- | CH₄ in Mole % | O₂ in Mole % | Time Min. | GHSV hr-1 | ECl ppmv |
|---|---|---|---|---|---|---|---|---|---|
| 750 | 25.2 | 58.9 | 14.9 | 4.0 | 9.8 | 5.0 | 7345 | 3097 | 0 |
| 750 | 25.9 | 63.3 | 16.2 | 3.9 | 9.9 | 5.0 | 8250 | 3097 | 0 |
| 750 | 24.4 | 63.1 | 15.4 | 3.9 | 9.9 | 4.9 | 8385 | 3097 | 0 |
| 750 | 33.3 | 53.8 | 17.9 | 6.3 | 9.9 | 4.9 | 8475 | 1935 | 0 |
| 750 | 33.3 | 53.7 | 17.9 | 6.3 | 9.9 | 4.9 | 8565 | 1935 | 0 |
| 750 | 33.8 | 53.1 | 17.9 | 6.4 | 9.9 | 4.9 | 8635 | 1935 | 0 |
| 750 | 37.1 | 50.5 | 18.6 | 7.4 | 9.9 | 4.9 | 8745 | 1548 | 0 |
| 750 | 37.6 | 49.7 | 18.5 | 7.4 | 9.9 | 4.9 | 8835 | 1548 | 0 |
| 750 | 36.0 | 50.8 | 18.3 | 7.3 | 9.9 | 4.9 | 9915 | 1548 | 0 |

EXAMPLES 20 TO 22

The following catalysts are prepared using the general procedures given below.

Procedure A: The selected alkaline earth metal component is dissolved in just enough deionized water at room temperature to wet the alpha-alumina support (The Norton Company, Akron, Ohio, SA-5402 30-60 mesh used for all catalysts). The alpha-alumina support is added to the solution and mixed to assure total uptake of the liquid and uniform impregnation of the alumina by incipient wetness. The alkaline earth metal component and alumina support are provided in relative amounts to each other to provide the desired loading of alkaline earth metal component on the catalyst. The material is then dried in a vacuum oven at about 110 to 130 °C. Drying is usually complete in 4 to 6 hours; however drying overnight does not appear to affect performance. The catalyst is tested without further treatment.

Procedure B: The selected insoluble alkaline earth metal component and the alpha-alumina support are added to 50 to 100 milliliters of deionized water at room temperature. The alkaline earth metal component and alpha-alumina support are provided in relative amounts to each other to provide the desired loading of alkaline earth metal component on the catalyst. The mixture is constantly stirred at

44

about 80 to 90 °C until a thick paste remains. The paste is then dried in a vacuum oven at about 110 to 130 °C. Drying is usually complete in 4 to 6 hours; however drying overnight does not appear to affect performance. The catalyst is tested without further treatment.

Procedure C: The selected metal component is dissolved in just enough deionized water at room temperature to wet the alkaline earth metal component/alpha-alumina catalyst prepared using procedure A or B. In the event that the metal component is not sufficiently soluble to form a solution, concentrated hydrochloric acid is added dropwise until the component dissolves. The alkaline earth metal component/alpha-alumina catalyst is added to the solution and mixed to assure total uptake of the liquid and uniform impregnation of the alumina by incipient wetness. The metal component and alkaline earth metal component/alpha-alumina catalyst are provided in relative amounts to each other to provide the desired loading of metal component on the catalyst. The material is then dried in a vacuum oven at about 110 to 130 °C. Drying is usually complete in 4 to 6 hours; however drying overnight does not appear to affect performance. The catalyst is tested without further treatment.

TABLE XXI

| Catalyst | Procedure | Dopant | Wt. % Based on Total Catalyst | Base Catalyst |
|---|---|---|---|---|
| B | A & C | $Co(NO_3)_2$ | 0.82 | 6.2 wt.% $SrCl_2$/alumina |
| H | B & C | $Co(NO_3)_2$ | 0.82 | 5.8 wt.% $SrCO_3$/alumina |

The following examples are conducted using the equipment described below. A quartz reactor is used which comprises a 1.5 centimeter (inside diameter) quartz tube about 55.9 centimeters in length with quartz "O"-ring joints at each end. At the bottom, a quartz effluent tube extends radially outward. Axially within the reactor tube is another quartz tube (1.3 centimeters outside diameter (1.1 centimeters inside diameter)) extending from the bottom (effluent end) of the reactor for about 28 centimeters. This tube is terminated with a joined 5 centimeters tube axially positioned thereon having an outside diameter of 0.5 centimeter and inside diameter of 0.3 centimeter. The annular region around this thinner tube ("annular reactor portion") receives the catalyst. These inner tubes form a thermocouple well. The thermocouple well extends 33 centimeters into the reactor from the bottom of the tube. The reactor is encased in a Lindberg oven for the mid-31 centimeters of its length. The incoming and exiting lines from the reactor are capable of being sampled by gas chromotography.

The catalyst bed is formed in the reactor by providing 20 to 40 mesh (U.S. Sieve Series) quartz chips around the larger diameter section of the thermocouple well, placing quartz wool over the chips (1 centimeter), forming the bed of catalysts (3 to 5 grams) wherein the catalyst particles have an average size of about 250 to 600 microns and then placing glass wool over the catalyst (1 centimeter) and either more quartz chips on the glass wool or a combination of an axially extending 1.3 centimeters outside diameter quartz solid rod with the quartz chips in the annular zone around the solid rod, to fill the upper portion of the reactor tube.

In the general operating procedure, the reactor is flushed with nitrogen while heating to about reaction temperature. The reactant stream is fed and the reactor is brought to the desired temperature. Periodic analyses of the gases are conducted (usually at intervals between one and two hours). The reactor pressure is about 5 pounds per square inch gauge (135 kPa absolute) and the feed usually contains $CH_4/O_2/N_2$ in a mole ratio, of about 2/1/20. In the following Tables =/-designates the ethylene to ethane mole ratio, and time is the time that the catalyst is on stream. "$CH_4$ Conv." is the total percent of methane reacted based on the amount of methane in the product gas. "$C_2$ Sel." is the mole percent of carbon converted to ethylene and ethane compared to the total moles of carbon in the observed products. "$C_3$ Sel." is the mole percent of carbon converted to propylene and propane. "$C_2$ yield" is the $CH_4$ Conv. times $C_2$ Sel./100. The gas hourly space velocity is based on the volumetric flow rate of the feed at ambient temperature and pressure per volume of the reactor occupied by the catalyst.

EXAMPLE 20 (Comparative)

The performance of catalyst 8 using the equipment and procedures described previously is presented in Tables XXII and XXIII. Reactant feed gas ratio of $CH_4/O_2/N_2$ is 2.2/1/20.

TABLE XXII

| Temp. °C. | $CH_4$ Conv. % | $C_2$Sel. % | $C_3$Sel. % | $C_2$Yield % | =/-Ratio molar | Time Hr. | GHSV $Hr^{-1}$ |
|---|---|---|---|---|---|---|---|
| 650 | 33 | 65 | 3.3 | 20.7 | 5.2 | 1 | 1500 |
| 650 | 24 | 66 | 3.2 | 15.4 | 2.3 | 3 | 1500 |
| 650 | 21 | 67 | 3.2 | 13.7 | 1.9 | 11 | 1500 |
| 650 | 20 | 68 | 3.8 | 13.2 | 1.7 | 19 | 1500 |
| 670 | 29 | 69 | 3.9 | 18.8 | 3.0 | 21 | 1500 |
| 670 | 25 | 69 | 3.8 | 16.9 | 3.0 | 23 | 1500 |
| 670 | 22 | 67 | 3.0 | 14.1 | 1.7 | 31 | 1500 |
| 670 | 20 | 56 | 2.9 | 10.6 | 1.2 | 35 | 1500 |

TABLE XXIII

| Temp. °C. | $CH_4$ Conv. % | $C_2$Sel. % | $C_3$Sel. % | $C_2$Yield % | =/-Ratio molar | Time Hr. | GHSV $Hr^{-1}$ |
|---|---|---|---|---|---|---|---|
| 600 | 17 | 44 | 1.3 | 7.7 | 2.4 | 1 | 612 |
| 600 | 13 | 44 | — | 6.1 | 1.5 | 3 | 612 |
| 600 | 11 | 45 | 1.0 | 4.9 | 1.4 | 7 | 612 |
| 625 | 20 | 56 | 2.3 | 11.6 | 2.5 | 9 | 612 |
| 625 | 19 | 56 | 2.2 | 10.6 | 2.2 | 13 | 612 |
| 625 | 16 | 58 | 2.1 | 9.6 | 1.8 | 21 | 612 |
| 650 | 27 | 61 | 3.2 | 17.5 | 4.1 | 23 | 612 |
| 650 | 29 | 65 | 3.1 | 19.5 | 4.5 | 27 | 612 |
| 650 | 27 | 64 | 4.8 | 17.9 | 3.5 | 33 | 612 |
| 650 | 24 | 67 | 3.2 | 16.2 | 3.0 | 45 | 612 |

EXAMPLE 21 (Comparative)

The performance of catalyst H using the equipment and procedures described previously is presented in Table XXIV. Reactant feed gas ratio of $CH_4/O_2/N_2$ is 2.2/1/20. No ethyl chloride is added.

TABLE XXIV

| Temp. °C. | $CH_4$ Conv. % | $C_2$Sel. % | $C_3$Sel. % | $C_2$Yield % | =/-Ratio molar | Time Hr. | GHSV $Hr^{-1}$ |
|---|---|---|---|---|---|---|---|
| 600 | 20 | 3.4 | 0.0 | 0.7 | 0.8 | 3 | 714 |
| 600 | 16 | 0.5 | 0.0 | 0.1 | 0.7 | 9 | 714 |
| 625 | 20 | 3.2 | 0.0 | 0.7 | 0.9 | 17 | 714 |
| 625 | 21 | 2.7 | 0.0 | 0.6 | 0.8 | 21 | 714 |
| 650 | 22 | 2.8 | 0.0 | 0.6 | 0.9 | 23 | 714 |
| 650 | 22 | 0.6 | 0.0 | 0.1 | 0.4 | 39 | 714 |

## EXAMPLE 22

The performance of catalyst H using the equipment and procedures described previously is presented in Table XXV. The results show the effect of ethyl chloride (ECl) in the feed stream on catalyst H. Reactant feed gas ratio of $CH_4/O_2/N_2$ is 2.2/1/20 with an ethyl chloride concentration of 100 ppm.

### TABLE XXV

| Temp. $\overline{C.}$ | $CH_4$ Conv. % | $C_2$ Sel. % | $C_2$ Sel. % | $C_2$ Yield % | =/-Ratio molar | Time Hr. | GHSV $Hr^{-1}$ |
|---|---|---|---|---|---|---|---|
| 650 | 13 | 8.5 | 0.0 | 1.1 | 0.7 | 1 | 880 |
| 650 | 13 | 12 | 0.0 | 1.6 | 0.8 | 5 | 880 |
| 650 | 12 | 18 | 0.2 | 2.2 | 0.9 | 13 | 880 |
| 650 | 12 | 25 | 0.4 | 3.0 | 1.1 | 17 | 880 |
| 700 | 23 | 54 | 2.3 | 12.5 | 2.0 | 21 | 880 |
| 700 | 18 | 51 | 3.3 | 9.5 | 1.7 | 27 | 880 |
| 700 | 14 | 43 | 1.3 | 6.4 | 1.6 | 39 | 880 |
| 650 | 6.0 | 37 | 0.9 | 2.3 | 1.0 | 43 | 880 |
| 650 | 6.1 | 39 | 0.7 | 2.4 | 1.0 | 51 | 880 |

## EXAMPLE 23

A catalyst of 3.6 weight percent barium oxide (BaO) on alpha aluminum oxide (a-alumina) is prepared by the general procedure B set forth above using 0.2914g of Johnson Matthey BaO, lot #16962, technical grade, 88-94% pure, and 7.5 g of Norton 5451 alpha aluminum oxide, lot #S96252B, 60/100 mesh. The resulting catalyst contains 0.00025 gram-atoms of barium per gram of support. In this procedure, BaO is mixed with 50 mL of deionized, distilled water. The mixture is stirred with the support, while heating at 70°C in a 100 mL glass beaker on a hotplate for 2 to 3 hours (or until almost no water is left), to distribute the material in and on the support. The resulting material is dried in a vacuum oven at 130°C under a vacuum of 16-84 kPa for about 18 to 20 hours, then calcined in air at 850°C for about 4 hours. The performance of the catalyst, using the equipment and procedures described above, with 5 grams of catalyst in Reactor D is reported in Table XXVI.

### TABLE XXVI

| Temp. $\overline{C}$ | $CH_4$ Conv. % | $C_2$ Sel % | $C_2$ Yield % | =/- | CH4 in Mole % | O2 in Mole % | Time hr | GHSV hr-l | ECl ppm |
|---|---|---|---|---|---|---|---|---|---|
| 750 | 32.8 | 59.7 | 19.3 | 2.5 | 9.9 | 4.9 | 51.5 | 2233 | 50 |
| 750 | 31.3 | 60.5 | 18.7 | 2.5 | 9.9 | 4.9 | 53.5 | 2233 | 50 |
| 750 | 31.3 | 60.4 | 18.4 | 2.6 | 10.1 | 4.9 | 56.5 | 2233 | 50 |
| 750 | 31.1 | 58.1 | 17.8 | 2.5 | 10.1 | 4.9 | 58.5 | 2233 | 50 |
| 750 | 29.9 | 58.8 | 17.4 | 2.4 | 10.1 | 4.9 | 60.5 | 2233 | 50 |
| 750 | 30.4 | 58.2 | 17.4 | 2.4 | 10.1 | 4.9 | 62.5 | 2233 | 50 |
| 750 | 29.2 | 59.3 | 17.1 | 2.3 | 10.1 | 4.9 | 64.5 | 2233 | 50 |
| 750 | 28.9 | 59.3 | 16.8 | 2.3 | 10.1 | 4.9 | 67.5 | 2233 | 50 |
| 750 | 29.1 | 61.0 | 17.2 | 2.3 | 10.1 | 4.9 | 69.5 | 2233 | 50 |
| 750 | 28.4 | 59.3 | 16.6 | 2.3 | 10.1 | 4.9 | 71.5 | 2233 | 50 |

## EXAMPLE 24

A catalyst of about 4.6 weight percent barium carbonate on α-alumina was prepared using 0.3759 g of Johnson Matthey barium carbonate ($BaCO_3$) powder, 99.997% pure, lot #S96252BR and 7.5 g of Norton 5451 α-Alumina, lot #S96252B, 60/100 mesh. The resulting catalyst contains about 0.00025 gram-atoms of barium per gram of support. In this procedure, $BaCO_3$ is mixed with 50 milliliters of deionized, distilled water. The mixture is stirred with the support, while heating at 70°C in a 100 milliliters glass beaker on a hotplate for 2 to 3 hours (or until almost no water is left), to distribute the material in and on the support. The resulting material is dried in a vacuum oven at 130°C under a vacuum of 16-84 kPa for about 18 to 20 hours, the calcined in air at 850°C for 4 hours. The performance of the catalyst, using the equipment and procedures described above, with 5 grams of catalyst in Reactor D is summarized in Table XXVII.

### TABLE XXVII

| Temp. °C | CH₄ Conv. % | C₂ Sel % | C₂ Yield % | =/- | CH4 in Mole % | O2 in Mole % | Time hr | GHSV hr-l | ECI ppm |
|---|---|---|---|---|---|---|---|---|---|
| 750 | 38.8 | 46.5 | 17.8 | 1.9 | 9.9 | 4.9 | 50.5 | 2233 | 50 |
| 750 | 37.8 | 47.5 | 17.8 | 1.9 | 9.9 | 4.9 | 52.5 | 2233 | 50 |
| 750 | 37.1 | 47.6 | 17.7 | 1.8 | 10.1 | 4.9 | 54.5 | 2233 | 50 |
| 750 | 36.9 | 48.4 | 17.7 | 1.8 | 10.1 | 4.9 | 57.5 | 2233 | 50 |
| 750 | 36.5 | 49.4 | 17.9 | 1.7 | 10.1 | 4.9 | 59.5 | 2233 | 50 |
| 750 | 35.8 | 50.6 | 17.9 | 1.6 | 10.1 | 4.9 | 61.5 | 2233 | 50 |
| 750 | 34.6 | 50.6 | 17.4 | 1.6 | 10.1 | 4.9 | 63.5 | 2233 | 50 |
| 750 | 33.4 | 52.1 | 17.3 | 1.5 | 10.1 | 4.9 | 65.5 | 2233 | 50 |
| 750 | 31.7 | 54.7 | 17.1 | 1.4 | 10.1 | 4.9 | 68.5 | 2233 | 50 |
| 750 | 30.3 | 55.2 | 16.6 | 1.3 | 10.1 | 4.9 | 70.5 | 2233 | 50 |

## EXAMPLE 25

A catalyst containing about 8.3 weight percent barium carbonate is prepared by the general procedure B set forth above using 1.0g Johnson Matthey barium carbonate ($BaCO_3$) powder, 99.99+% pure, lot #S97090R and 10 g of Johnson Matthey titanium dioxide ($TiO_2$), 30/60 mesh, 0.82 $m^2$/g surface area, lot #S96579, 99.995% pure. In this procedure, $BaCO_3$ is mixed with 40 mL of deionized, distilled water. The mixture is stirred with the support, while heating at 70°C in a glass container on a hotplate for 2 to 3 hours (or until almost no water is left), to distribute the material in and on the support. The resulting material is dried in a vacuum oven at 130°C under a vacuum of 16-84 kPa for about 18 to 20 hours, then calcined in air at 850°C for 4 hours. The performance of the catalyst using the equipment and procedures described above, with 5 grams of catalyst in Reactor D is summarized in Table XXVIII.

### TABLE XXVIII

| Temp. °C | CH₄ Conv. % | C₂ Sel % | C₂ Yield % | =/- | CH4 in Mole % | O2 in Mole % | Time hr | GHSV hr-l | ECI ppm |
|---|---|---|---|---|---|---|---|---|---|
| 750 | 29.0 | 64.2 | 18.0 | 5.0 | 10.4 | 5.0 | 170 | 2226 | 500 |
| 750 | 28.9 | 63.9 | 17.9 | 5.1 | 10.4 | 5.0 | 202 | 2226 | 500 |
| 750 | 28.9 | 63.9 | 18.1 | 5.2 | 10.4 | 5.0 | 104 | 2226 | 500 |
| 750 | 29.1 | 64.0 | 18.1 | 4.8 | 10.3 | 5.0 | 272 | 2226 | 500 |
| 750 | 34.8 | 60.7 | 20.1 | 8.2 | 10.8 | 6.1 | 296 | 2226 | 500 |

## EXAMPLE 26

A catalyst of 7.0 weight percent hydrated barium hydroxide (Ba(OH)$_2$·8H$_2$O) on titanium dioxide (TiO$_2$) is prepared by the general procedure A set forth above using 0.4808 g of Baker Analyzed Reagent barium hydroxide (Ba(OH)$_2$·8H$_2$O), lot #514157 and 6 g of Johnson Matthey titanium dioxide (TiO$_2$), 30/60 mesh,0.82 m$^2$/g surface area, lot #S96579, 99.995% pure. The catalyst contains about 0.00025 gram-atoms of barium per gram of support. In this procedure, the incipient wetness technique is used, in which Ba(OH)$_2$ is dissolved in 1.02 g of deionized, distilled water. The solution is then added to 6 g of TiO$_2$ and just fills the pores of the support. The resulting material is dried in a vacuum oven at 130°C under a vacuum of 16-84 kPa for about 18-20 hours, then calcined in air at 850°C for 4 hours. The performance of the catalyst, using the equipment and procedures described above, with 5 grams of catalyst in Reactor D is reported in Table XXIX.

TABLE XXIX

| Temp. °C | CH$_4$ Conv. % | C$_2$ Sel % | C$_2$ Yield % | =/- | CH4 in Mole % | O2 in Mole % | Time hr | GHSV hr-l | ECl ppm |
|---|---|---|---|---|---|---|---|---|---|
| 750 | 27.2 | 70.8 | 18.7 | 4.3 | 10.4 | 5.0 | 2.5 | 2229 | 500 |
| 750 | 27.5 | 67.2 | 18.1 | 4.5 | 10.3 | 5.0 | 9.0 | 2229 | 500 |
| 750 | 27.4 | 65.7 | 17.6 | 5.0 | 10.3 | 5.0 | 50.0 | 2229 | 500 |
| 750 | 28.9 | 65.7 | 18.2 | 4.6 | 10.5 | 5.0 | 78.0 | 2229 | 500 |
| 750 | 28.1 | 65.7 | 17.9 | 4.6 | 10.3 | 5.0 | 100.0 | 2229 | 500 |

## EXAMPLE 27

Johnson Matthey Technical Grade BaO is heated to 850°C in air in a zirconium crucible for 4 hours, then meshed to 30/60 mesh. It is tested at 750°C, 2230 hr-1 GHSV with 500 ppm ethyl chloride, 10% methane, 5% oxygen, and balance nitrogen in the feed stream. At times onstream between 45 and 85 hours, the selectivity to C$_2$ products was between 52 and 55 percent, the methane conversion was between 15 and 18 percent, and the C$_2$ yield was between 5 and 10 percent. The ethylene to ethane ratio was below 2.

**Claims**

1. A process for oxidative coupling of alkane of 1 to 3 carbon atoms to heavier hydrocarbon comprising feeding the alkane and reactive oxygen-containing material and halogen-containing vapor phase additive in an amount sufficient to enhance at least one of the conversion of alkane and selectivity to higher hydrocarbon, to a reaction zone containing a catalytically effective amount of oxidative coupling catalyst said oxidative coupling catalyst comprising a catalytically active amount of at least one of barium hydroxide, strontium hydroxide, barium carbonate and strontium carbonate on a support; maintaining the reaction zone under oxidative coupling conditions to convert at least a portion of the alkane to heavier hydrocarbon, and withdrawing from the reaction zone an effluent containing heavier hydrocarbon produced in the reaction zone.

2. The process of claim 1 wherein the reactive oxygen-containing material comprises oxygen.

3. The process of claim 2 wherein sufficient barium hydroxide, strontium hydroxide, barium carbonate and strontium carbonate is present to provide a yield of higher hydrocarbon based on alkane feed of at least 15 mole percent under Standard Reference Conditions.

4. The process of claim 2 wherein the total barium hydroxide, strontium hydroxide, barium carbonate and strontium carbonate compound comprises 1 to 20 weight percent of the catalyst.

5. The process of claim 4 wherein the catalyst comprises at least one of barium carbonate and strontium carbonate.

6. The process of claim 4 wherein the catalyst comprises at least one of barium hydroxide and strontium hydroxide.

7. The process of claim 6 wherein the barium hydroxide or strontium hydroxide comprises hydrated barium oxide or hydrated strontium oxide.

8. The process of claim 4 wherein the support comprises alumina.

9. The process of claim 8 wherein the support has a surface area of at least about 0.2 square meter per gram.

10. The process of claim 1 wherein the support has a surface area of about 0.2 to 60 square meters per gram.

11. The process of claim 3 wherein the halogen-containing vapor phase additive comprises at least one compound containing chlorine, bromine or iodine.

12. The process of claim 11 wherein the halogen-containing vapor phase additive is provided in an amount of about 1 to 1000 ppmv based on the volume of feed to the reaction zone.

13. The process of claim 12 wherein the halogen-containing vapor phase additive contains chlorine.

14. The process of claim 12 wherein the oxidative coupling conditions comprise a temperature in the range of about 600°C to 800°C.

15. The process of claim 14 wherein the oxidstive coupling conditions comprise a gas hourly space velocity between about 500 and 15000 reciprocal hours.

16. The process of claim 15 wherein sufficient barium carbonate and/or strontium carbonate is present to provide a yield of higher hydrocarbon based on alkane feed of at least 15 mole percent under Standard Reference Conditions.

17. The process of claim 1 wherein the halogen-containing vapor phase additive comprises at least one of hydrogen halide, organic halide of 1 to 3 carbon atoms and halogen, wherein the halide or halogen is at least one of chlorine, bromine and iodine.

18. The process of claim 1 wherein the mole ratio of alkane to oxygen atom is about 1:1 to 20:1.

19. The process of claim 16 wherein the mole ratio of alkane to oxygen atom is about 1:1 to 20:1.

20. A catalyst comprising at least one of barium hydroxide strontium hydroxide, barium carbonate and strontium carbonate on a support which has been subjected to the process of claim 1.

21. The catalyst of claim 20 which when prepared comprises about 1 to 20 weight percent barium hydroxide, strontium hydroxide, barium carbonate and strontium carbonate.

22. The catalyst of claim 20 which comprises an alumina support.

23. The catalyst of claim 22 in which the alumina is alpha alumina.

24. A process for activating a catalyst comprising at least one of barium hydroxide, strontium hydroxide, barium carbonate and strontium on a support comprising subjecting the catalyst to the process of claim 1 for a time sufficient to increase the yield of alkane to heavier hydrocarbon in the process.

25. The process of claim 24 wherein the duration of the activation is sufficient to increase the yield of alkane to heavier hydrocarbon by at least about 50 percent.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 108, no. 21, May 1988, page 648, abstract no. 186163g, Columbus, Ohio, US; & JP-A-62 267 243 (IDEMITSU KOSAN CO., LTD) 19-11-1987 --- | 1-3,5,20, 24 | C 07 C 2/84 B 01 J 23/02 B 01 J 27/232 |
| X | DE-A-3 534 530 (M. BAERNS) * Claims * --- | 1,5-6,8, 14,18-22, 24 | |
| X | US-A-4 465 893 (OLAH) * Claims; examples * ----- | 1-2,11,13 | |
| | | | **TECHNICAL FIELDS SEARCHED (Int. Cl.5)** |
| | | | C 07 C 2/00 |

The present search report has been drawn up for all claims

| Place of search | Date of completion of search | Examiner |
|---|---|---|
| The Hague | 27 November 90 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document